(19) Europäisches Patentamt
European Patent Office
Office européen des brevets

(11) Veröffentlichungsnummer : **0 407 891 B1**

(12) **EUROPÄISCHE PATENTSCHRIFT**

(45) Veröffentlichungstag der Patentschrift : **23.06.93 Patentblatt 93/25**

(51) Int. Cl.$^5$ : **C07C 251/48,** A01N 37/50, C07C 69/738

(21) Anmeldenummer : **90112835.5**

(22) Anmeldetag : **05.07.90**

# (54) Neue 3-Methoximinopropionsäureester und diese enthaltende Fungizide.

(30) Priorität : **13.07.89 DE 3923093**

(43) Veröffentlichungstag der Anmeldung : **16.01.91 Patentblatt 91/03**

(45) Bekanntmachung des Hinweises auf die Patenterteilung : **23.06.93 Patentblatt 93/25**

(84) Benannte Vertragsstaaten : **AT BE CH DE DK ES FR GB GR IT LI NL SE**

(56) Entgegenhaltungen :
**EP-A- 0 253 213**
**EP-A- 0 254 426**

(73) Patentinhaber : **BASF Aktiengesellschaft**
**Carl-Bosch-Strasse 38**
**W-6700 Ludwigshafen (DE)**

(72) Erfinder : **Wenderoth, Bernd, Dr.**
**Schwalbenstrasse 26**
**W-6840 Lampertheim (DE)**
Erfinder : **Sauter, Hubert, Dr.**
**Neckarpromenade 20**
**W-6800 Mannheim 1 (DE)**
Erfinder : **Roehl, Franz, Dr.**
**Karl-Otto-Braum-Strasse 8**
**W-6700 Ludwigshafen (DE)**
Erfinder : **Ammermann, Eberhard, Dr.**
**Sachsenstrasse 3**
**W-6700 Ludvigshafen (DE)**
Erfinder : **Lorenz, Gisela, Dr.**
**Erlenweg 13**
**W-6730 Neustadt (DE)**

EP 0 407 891 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft neue 3-Methoximinopropionsäureester und diese enthaltende Fungizide. Es ist bekannt, Oximetherderivate wie zum Beispiel 2-(2-Methylbenzyloxy)-phenylglyoxylsäuremethylester-O-methyloxim als Fungizide zu benutzen (EP 253213). Ihre Wirkung ist jedoch in manchen Indikationen oft nicht ausreichend.

Es wurde nun gefunden, daß neue 3-Methoximinopropionsäureester der allgemeinen Formel I

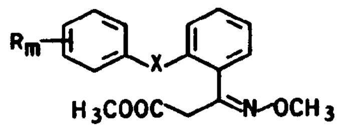

(I)

in der
R (m = 1 bis 5) gleiche oder verschiedene Substituenten Wasserstoff, Halogen, Cyano, Nitro, $C_1$-$C_{15}$-Alkyl, $C_3$-$C_6$-Cycloalkyl, $C_3$-$C_6$-Alkenyl, $C_1$-$C_4$-Alkoxy, $C_1$-$C_2$-Halogenalkyl, $C_1$-$C_2$-Halogenalkoxy, gegebenenfalls substituiertes Phenyl, gegebenenfalls substituiertes Phenoxy, gegebenenfalls substituiertes Benzyl oder gegebenenfalls substituiertes Benzyloxy bedeutet, oder in der die Gruppe

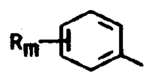

α-Naphthyl oder β-Naphthyl und
X
Methylenoxy, Oxymethylen, Ethylen, Ethenylen, Thiomethylen oder Sauerstoff bedeutet, eine ausgezeichnete fungizide Wirkung besitzen, die besser ist als die der bekannten Oximetherderivate.

Die in der allgemeinen Formel I aufgeführten Reste können beispielsweise folgende Bedeutungen haben:
R (m = 1 bis 5) kann zum Beispiel bedeuten:

Wasserstoff, Halogen (z.B. Fluor, Chlor, Brom, Jod), Cyano, Nitro, $C_1$-$C_{15}$-Alkyl, (z.B. Methyl, Ethyl, n- oder i-Propyl; n-, i-, s-oder t-Butyl; Pentyl, Hexyl, Heptyl, Octyl, Nonyl, Decyl), $C_3$-$C_6$-Cycloalkyl (z.B. Cyclopropyl, Cyclopentyl, Cyclohexyl), $C_3$-$C_6$-Alkenyl (z.B. 1-Propenyl, 2-Propenyl), $C_1$-$C_4$-Alkoxy (z.B. Methoxy, Ethoxy, n-Propoxy, i-Propoxy, n-Butoxy, t-Butoxy), $C_1$-$C_2$-Halogenalkyl (z.B. Difluormethyl, Trifluormethyl, Chlormethyl, Dichlormethyl, Trichlormethyl, Pentafluorethyl), $C_1$-$C_2$-Halogenalkoxy (z.B. Trifluormethoxy, Difluormethoxy, Tetrafluorethoxy, Pentafluorethoxy), gegebenenfalls substituiertes Phenyl (z.B. Phenyl, $C_1$-$C_4$ Alkylphenyl, Halogenphenyl), gegebenenfalls substituiertes Phenoxy (z.B. Phenoxy, $C_1$-$C_4$-Alkylphenoxy, Halogenphenoxy), gegebenenfalls substiutiertes Benzyl (z.B. Benzyl, Halogenbenzyl), gegebenenfalls substituiertes Benzyloxy (z.B. Benzyloxy, Halogenbenzyloxy, $C_1$-$C_4$-Alkylbenzyloxy). Außerdem kann die Gruppe

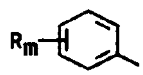

für α-Naphthyl oder β-Naphthyl stehen.
X
ist bevorzugt eine $-CH_2O-$, $-OCH_2$, $-CH_2$-$CH_2-$, -CH=CH-, $-SCH_2$-Kette oder steht für O.
m
bedeutet 1, 2, 3, 4 oder 5. 1 bis 3 wird bevorzugt.

Die neuen Verbindungen der allgemeinen Formel I können aufgrund der C=N-Doppelbindung sowohl als E- als auch als Z-Isomere vorliegen. Sowohl die einzelnen isomeren Verbindungen als auch ihre Gemische werden von der Erfindung umfaßt und sind als Fungizide brauchbar.

Die neuen Verbindungen der allgemeinen Formel I können beispielsweise nach folgenden Verfahren hergestellt werden.

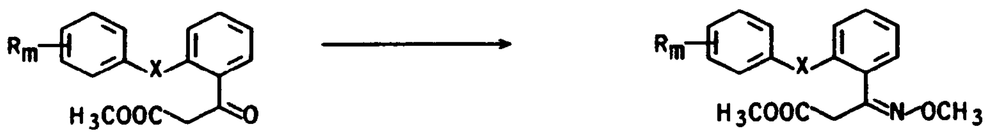

(II) (I)

Die β-Ketoester der allgemeinen Formel II können in die neuen Verbindungen der allgemeinen Formel I überführt werden, indem man sie mit O-Methylhydroxylamin umsetzt (vgl. z.B. H.V. Secor, E.B. Sanders, J. Org. Chem. 43, 2539-2541 (1978)), oder indem man sie zuerst mit Hydroxylamin zum entsprechenden Oxim und danach mit einem Methylierungsmittel wie z.B. Methyljodid oder Dimethylsulfat umsetzt (vgl. auch EP 253213).

Die β-Ketoester der allgemeinen Struktur II sind neue und wertvolle Zwischenprodukte, die von dieser Erfindung ebenfalls erfaßt werden.

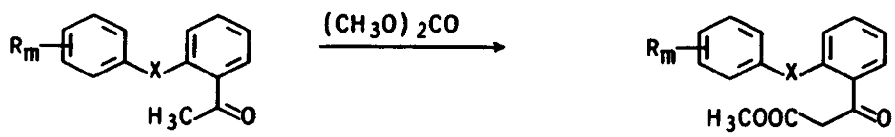


(III) (II)

Sie lassen sich nach an sich bekannten Verfahren (vgl. F.W. Swamer, Ch.R. Hauser, J. Am. Chem. Soc. 72, 1352-1356 (1950)) herstellen, indem man ein Acetophenon der allgemeinen Formel III in einem geeigneten Lösungsmittel wie zum Beispiel Diethylether mit Dimethylcarbonat in Gegenwart einer geeigneten Base wie zum Beispiel Natriumhydrid oder Natriummethanolat umsetzt.

Acetophenonderivate der allgemeinen Formel IIIa ($X=CH_2O$) lassen sich auf übliche Art und Weise herstellen, indem man Benzylhalogenide der allgemeinen Formel IV, wobei Hal für Halogen steht, und $R_m$ die oben genannte Bedeutung hat, mit 2-Hydroxyacetophenon in Gegenwart einer Base wie zum Beispiel Natriumcarbonat in Gegenwart eines geeigneten Lösungsmittels wie zum Beispiel Methanol umsetzt.

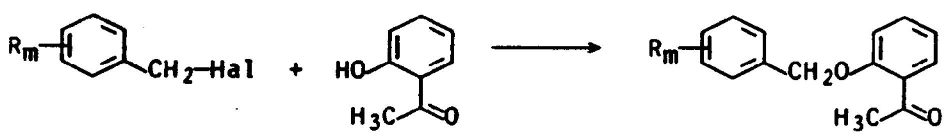

(IV) (IIIa)

Acetophenonderivate der allgemeinen Formel IIIb ($X=OCH_2$) lassen sich auf übliche Art und Weise herstellen, indem man Phenole der allgemeinen Formel V, wobei $R_m$ die obengenannte Bedeutung hat, mit 2-Cyanobenzylbromid in Gegenwart einer Base wie zum Beispiel Natriumcarbonat in Gegenwart eines geeigneten Lösungsmittels wie zum Beispiel Methanol umsetzt.

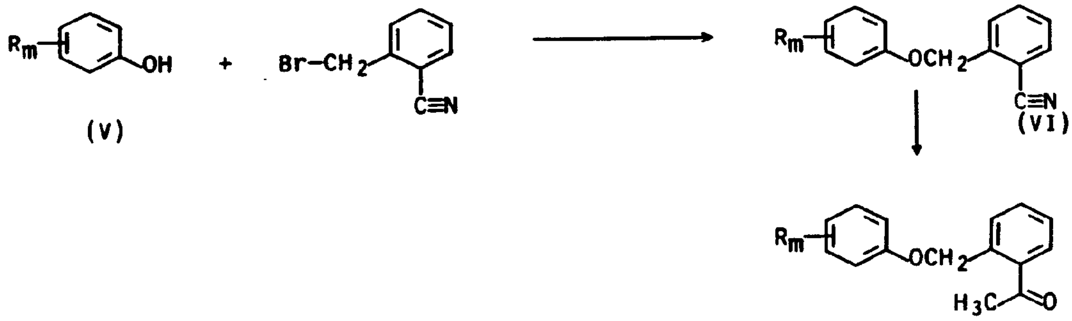

(V) (VI)

(IIIb)

Das erhaltene Nitril der allgemeinen Formel VI läßt sich in einer Grignard-Reaktion mit Methylmagnesiumhalogenid in das Acetophenon IIIb überführen (vgl. E.C. Ashby et al., J. Am. Chem. Soc. 95, 4896, 5186 (1973)).

Geht man von den analogen Thiophenolen der allgemeinen Formel VII aus, so erhält man auf gleichem Weg die Acetophenonderivate der allgemeinen Formel IIIc ($X=SCH_2$)

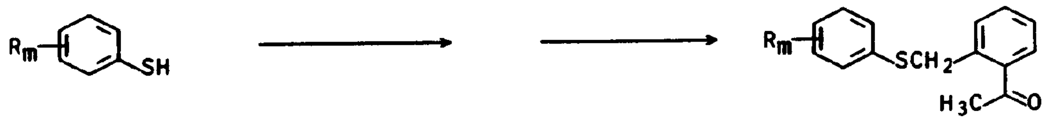

(VII) (IIIc)

Acetophenonderivate der allgemeinen Formel IIId (X = CH=CH) lassen sich herstellen, indem man auf bekannte Weise in einer Wittig-Reaktion (vgl. G. Wittig, U. Schöllkopf, Org. Synth. Coll. Vol. V, 751 (1973)) 2-Cyanobenzaldehyd mit Benzylphosphoniumhalogeniden der allgemeinen Formel VIII umsetzt, wobei $R_m$ die obengenannte Bedeutung hat.

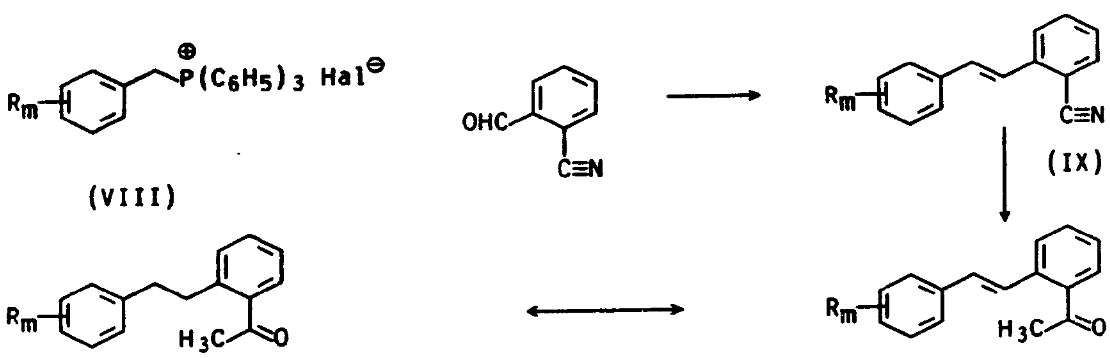

(VIII) (IX)

(IIIe) (IIId)

Das erhaltene Nitril der allgemeinen Formel IX, läßt sich in einer Grignard-Reaktion mit Methylmagnesiumhalogenid in das Acetophenon IIId überführen. (vgl. E.C. Ashby et al, J. Am. Chem. Soc. 95, 4896, 5186 (1973)).

Durch Reduktion der Doppelbindung entweder katalytisch mit Wasserstoff (vgl. Houben-Weyl, Methoden der organischen Chemie V/2b, 264-267 (1981)) oder mit Diimin (vgl. E.E. van Tamelen et al, J. Am. Chem. Soc. 83, 4302 (1961) lassen sich daraus die Acetophenonderivate der allgemeinen Formel IIIe ($X = CH_2$-$CH_2$) herstellen.

Acetophenonderivate der allgemeinen Formel IIIf (X=O) lassen sich zum Beispiel in bekannter Weise herstellen (T.W. Harris et al, J. Med. Chem. 25, 855-858 (1982)), indem man 2-Chloracetophenon in Gegenwart von Kupferpulver und Kaliumcarbonat mit Phenolen der Formel V umsetzt.

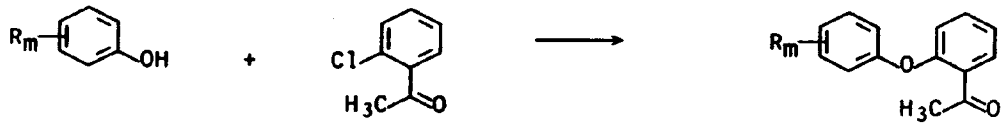

(V) (IIIf)

Die Herstellung der neuen erfindungsgemäßen Verbindungen wird durch folgende Beispiele erläutert:

Vorschrift 1:

2-(2-Methylbenzyloxy)-acetophenon

In 500 ml absolutem Ethanol werden 102 g (0,75 mol) 2-Hydroxyacetophenon und 102 g (0,75 mol) Kaliumcarbonat vorgelegt. Dazu tropft man 139 g (0,75 mol) 2-Methylbenzylbromid und erhitzt 10 h auf Rückflußtemperatur. Nach dem Abkühlen wird abgesaugt und das Filtrat eingeengt. Das Rohprodukt wird aus Diethylether umkristallisiert. Man erhält 106 g (59 %) als Kristalle (Fp = 53 - 55°C).

Vorschrift 2:

3-[2-(2-Methylbenzyloxy)-phenyl]-3-oxo-propionsäuremethylester

In 225 ml absolutem Diethylether werden unter Stickstoff 14 g (0,6 mol) Natriumhydrid (100 %ig) und 54 g (0,6 mol) Dimethylcarbonat vorgelegt. Dazu tropft man bei 20-35°C 72 g (0,3 mol) 2-(2-Methylbenzyloxy)acetophenon, gelöst in 300 ml absolutem Ether, und erhitzt 5 h unter Rückfluß. Bei 10°C werden zuerst 45 ml Methanol und danach 100 ml $H_2O$ langsam zugetropft. Mit verdünnter Salzsäure wird dann auf pH 7 eingestellt. Die organische Phase wird mehrmals mit $H_2O$ gewaschen, über Natriumsulfat getrocknet und eingeengt. Man erhält das Produkt in quantitativer Ausbeute als Kristalle (Fp=38-42°C).
$^1$H-NMR: $\delta$ = 2.39(s,3H); 3.55(s,2H); 5.15(s,2H), 7.0 - 7.9(m,8H).

Herstellungsbeispiel 1

3-[2-(2-Methylbenzyloxy)-phenyl]-3-methoximinopropionsäure-methylester (Verbindung Nr. 66)

In 250 ml Pyridin werden 14,9 g (0,05 mol) 3-[2-(2-Methylbenzyloxy)-phenyl]-3-oxo-propionsäuremethylester und 8,35 g (0,1 mol) O-Methylhydroxylaminhydrochlorid vorgelegt und 18 h bei 70°C gerührt. Anschließend wird im Vakuum eingeengt. Der Rückstand wird in Essigester aufgenommen, gründlich mit $H_2O$ gewaschen, über Natriumsulfat getrocknet und erneut eingeengt. Man erhält 12 g (73 %) des gewünschten Esters als Öl (Isomerenverhältnis 85/15).
$^1$H-NMR ($CDCl_3$): $\delta$ = 2.35(m,3H); 3.30/3.59(2xs,3H);
3.56/3.75(2xs,2H); 3.85/3.96(2xs,3H), 5.05(s,2H); 6.9-7.5(m,8H).

In entsprechender Weise können die folgenden Verbindungen hergestellt werden.

Tabelle 1

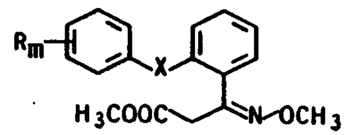

(I)

| Nr. | $R_m$ | X | Fp(°C) | IR($cm^{-1}$) |
|---|---|---|---|---|
| 1 | H | $CH_2O$ | | |
| 2 | 2-F | $CH_2O$ | | |
| 3 | 3-F | $CH_2O$ | | |
| 4 | 4-F | $CH_2O$ | öl | 2940, 1741, 1512, 1226, 1050, 754 |
| 5 | 2,3-$F_2$ | $CH_2O$ | | |
| 6 | 2,4-$F_2$ | $CH_2O$ | | |
| 7 | 2,4,6-$F_3$ | $CH_2O$ | | |
| 8 | 2,3,4,5,6-$F_5$ | $CH_2O$ | | |
| 9 | 2-Cl | $CH_2O$ | | |
| 10 | 3-Cl | $CH_2O$ | | |
| 11 | 4-Cl | $CH_2O$ | öl | 2940, 1741, 1494, 1234, 1050, 754 |
| 12 | 2,3-$Cl_2$ | $CH_2O$ | | |
| 13 | 2,4-$Cl_2$ | $CH_2O$ | | |
| 14 | 2,5-$Cl_2$ | $CH_2O$ | | |
| 15 | 2,6-$Cl_2$ | $CH_2O$ | | |

| Nr. | $R_m$ | X | Fp(°C) | IR($cm^{-1}$) |
|---|---|---|---|---|
| 16 | 3,4-$Cl_2$ | $CH_2O$ | | |
| 17 | 3,5-$Cl_2$ | $CH_2O$ | | |
| 18 | 2,3,4-$Cl_3$ | $CH_2O$ | | |
| 19 | 2,3,5-$Cl_3$ | $CH_2O$ | | |
| 20 | 2,3,6-$Cl_3$ | $CH_2O$ | | |
| 21 | 2,4,5-$Cl_3$ | $CH_2O$ | | |
| 22 | 2,4,6-$Cl_3$ | $CH_2O$ | | |
| 23 | 3,4,5-$Cl_3$ | $CH_2O$ | | |
| 24 | 2,3,4,6-$Cl_4$ | $CH_2O$ | | |
| 25 | 2,3,5,6-$Cl_4$ | $CH_2O$ | | |
| 26 | 2,3,4,5,6-$Cl_5$ | $CH_2O$ | | |
| 27 | 2-Br | $CH_2O$ | | |
| 28 | 3-Br | $CH_2O$ | | |
| 29 | 4-Br | $CH_2O$ | 91-95 | 2940,1746,1447,1176,1044,751 |
| 30 | 2,4-$Br_2$ | $CH_2O$ | | |
| 31 | 2,5-$Br_2$ | $CH_2O$ | | |
| 32 | 2,6-$Br_2$ | $CH_2O$ | | |
| 33 | 2,4,6-$Br_3$ | $CH_2O$ | | |
| 34 | 2,3,4,5,6-$Br_5$ | $CH_2O$ | | |
| 35 | 2-J | $CH_2O$ | | |
| 36 | 3-J | $CH_2O$ | | |
| 37 | 4-J | $CH_2O$ | | |
| 38 | 2,4-$J_2$ | $CH_2O$ | | |
| 39 | 2-Cl, 3-F | $CH_2O$ | | |
| 40 | 2-Cl, 4-F | $CH_2O$ | | |
| 41 | 2-Cl, 5-F | $CH_2O$ | | |

| Nr. | $R_m$ | X | Fp(°C) | $IR(cm^{-1})$ |
|---|---|---|---|---|
| 42 | 2-Cl, 6-F | $CH_2O$ | | |
| 43 | 2-Cl, 3-Br | $CH_2O$ | | |
| 44 | 2-Cl, 4-Br | $CH_2O$ | | |
| 45 | 2-Cl, 5-Br | $CH_2O$ | | |
| 46 | 2-Cl, 6-Br | $CH_2O$ | | |
| 47 | 2-Br, 3-Cl | $CH_2O$ | | |
| 48 | 2-Br, 4-Cl | $CH_2O$ | | |
| 49 | 2-Br, 5-Cl | $CH_2O$ | | |
| 50 | 2-Br, 3-F | $CH_2O$ | | |
| 51 | 2-Br, 4-F | $CH_2O$ | | |
| 52 | 2-Br, 5-F | $CH_2O$ | | |
| 53 | 2-Br, 6-F | $CH_2O$ | | |
| 54 | 2-F, 3-Cl | $CH_2O$ | | |
| 55 | 2-F, 4-Cl | $CH_2O$ | | |
| 56 | 2-F, 5-Cl | $CH_2O$ | | |
| 57 | 3-Cl, 4-F | $CH_2O$ | | |
| 58 | 3-Cl, 5-F | $CH_2O$ | | |
| 59 | 3-Cl, 4-Br | $CH_2O$ | | |
| 60 | 3-Cl, 5-Br | $CH_2O$ | | |
| 61 | 3-F, 4-Cl | $CH_2O$ | | |
| 62 | 3-F, 4-Br | $CH_2O$ | | |
| 63 | 3-Br, 4-Cl | $CH_2O$ | | |
| 64 | 3-Br, 4-F | $CH_2O$ | | |
| 65 | 2,6-$Cl_2$, 4-Br | $CH_2O$ | | |
| 66 | 2-$CH_3$ | $CH_2O$ | Öl | 2940, 1742, 1450, 1231, 1050, 751 |
| 67 | 3-$CH_3$ | $CH_2O$ | | |

| Nr. | $R_m$ | X | Fp(°C) | IR($cm^{-1}$) |
|---|---|---|---|---|
| 68 | $4-CH_3$ | $CH_2O$ | | |
| 69 | $2,3-(CH_3)_2$ | $CH_2O$ | | |
| 70 | $2,4-(CH_3)_2$ | $CH_2O$ | | |
| 71 | $2,5-(CH_3)_2$ | $CH_2O$ | | |
| 72 | $2,6-(CH_3)_2$ | $CH_2O$ | | |
| 73 | $3,4-(CH_3)_2$ | $CH_2O$ | | |
| 74 | $3,5-(CH_3)_2$ | $CH_2O$ | | |
| 75 | $2,3,4-(CH_3)_3$ | $CH_2O$ | | |
| 76 | $2,3,5-(CH_3)_3$ | $CH_2O$ | | |
| 77 | $2,3,6-(CH_3)_3$ | $CH_2O$ | | |
| 78 | $2,4,5-(CH_3)_3$ | $CH_2O$ | | |
| 79 | $2,4,6-(CH_3)_3$ | $CH_2O$ | | |
| 80 | $3,4,5-(CH_3)_3$ | $CH_2O$ | | |
| 81 | $2,3,4,6-(CH_3)_4$ | $CH_2O$ | | |
| 82 | $2,3,5,6-(CH_3)_4$ | $CH_2O$ | | |
| 83 | $2,3,4,5,6-(CH_3)_5$ | $CH_2O$ | | |
| 84 | $2-C_2H_5$ | $CH_2O$ | | |
| 85 | $3-C_2H_5$ | $CH_2O$ | | |
| 86 | $4-C_2H_5$ | $CH_2O$ | | |
| 87 | $2,4-(C_2H_5)_2$ | $CH_2O$ | | |
| 88 | $2,6-(C_2H_5)_2$ | $CH_2O$ | | |
| 89 | $3,5-(C_2H_5)_2$ | $CH_2O$ | | |
| 90 | $2,4,6-(C_2H_5)_3$ | $CH_2O$ | | |
| 91 | $2-n-C_3H_7$ | $CH_2O$ | | |
| 92 | $3-n-C_3H_7$ | $CH_2O$ | | |
| 93 | $4-n-C_3H_7$ | $CH_2O$ | | |

| Nr. | $R_m$ | X | Fp(°C) | IR($cm^{-1}$) |
|---|---|---|---|---|
| 94 | 2-i-$C_2H_7$ | $CH_2O$ | | |
| 95 | 3-i-$C_2H_7$ | $CH_2O$ | | |
| 96 | 4-i-$C_3H_7$ | $CH_2O$ | | |
| 97 | 2,4-(i-$C_3H_7$)$_2$ | $CH_2O$ | | |
| 98 | 2,6-(i-$C_3H_7$)$_2$ | $CH_2O$ | | |
| 99 | 3,5-(i-$C_3H_7$)$_2$ | $CH_2O$ | | |
| 100 | 2,4,6-(i-$C_3H_7$)$_3$ | $CH_2O$ | | |
| 101 | 2-s-$C_4H_9$ | $CH_2O$ | | |
| 102 | 3-s-$C_4H_9$ | $CH_2O$ | | |
| 103 | 4-s-$C_4H_9$ | $CH_2O$ | | |
| 104 | 2-t-$C_4H_9$ | $CH_2O$ | | |
| 105 | 3-t-$C_4H_9$ | $CH_2O$ | | |
| 106 | 4-t-$C_4H_9$ | $CH_2O$ | | |
| 107 | 2,3-(t-$C_4H_9$)$_2$ | $CH_2O$ | | |
| 108 | 2,4-(t-$C_4H_9$)$_2$ | $CH_2O$ | | |
| 109 | 2,5-(t-$C_4H_9$)$_2$ | $CH_2O$ | | |
| 110 | 2,6-(t-$C_4H_9$)$_2$ | $CH_2O$ | | |
| 111 | 3,5-(t-$C_4H_9$)$_2$ | $CH_2O$ | | |
| 112 | 2,4,6-(t-$C_4H_9$)$_3$ | $CH_2O$ | | |
| 113 | 4-n-$C_9H_{19}$ | $CH_2O$ | | |
| 114 | 4-n-$C_{12}H_{25}$ | $CH_2O$ | | |
| 115 | 3-n-$C_{15}H_{31}$ | $CH_2O$ | | |
| 116 | 4-(1,1,3,3,-Tetramethylbutyl) | $CH_2O$ | | |
| 117 | 4-(1,1,3,-Trimethylbutyl) | $CH_2O$ | | |
| 118 | 2-t-$C_4H_9$, 4-$CH_3$ | $CH_2O$ | | |
| 119 | 2-t-$C_4H_9$, 5-$CH_3$ | $CH_2O$ | | |

| Nr. | $R_m$ | X | Fp(°C) | IR($cm^{-1}$) |
|---|---|---|---|---|
| 120 | 2,6-$(t-C_4H_9)_2$, 4-$CH_3$ | $CH_2O$ | | |
| 121 | 2-$CH_3$, 4-t-$C_4H_9$ | $CH_2O$ | | |
| 122 | 2-$CH_3$, 6-t-$C_4H_9$ | $CH_2O$ | | |
| 123 | 2-$CH_3$, 4-i-$C_3H_7$ | $CH_2O$ | | |
| 124 | 2-$CH_3$, 5-i-$C_3H_7$ | $CH_2O$ | | |
| 125 | 3-$CH_3$, 4-i-$C_3H_7$ | $CH_2O$ | | |
| 126 | 2-i-$C_3H_7$, 5-$CH_3$ | $CH_2O$ | | |
| 127 | 2,4-$(t-C_4H_9)_2$, 6-i-$C_3H_7$ | $CH_2O$ | | |
| 128 | 2-$C_3H_5$ (=Allyl) | $CH_2O$ | | |
| 129 | 3-$C_3H_5$ | $CH_2O$ | | |
| 130 | 4-$C_3H_5$ | $CH_2O$ | | |
| 131 | 2-$C_3H_5$, 6-$CH_3$ | $CH_2O$ | | |
| 132 | 2-cyclo-$C_6H_{11}$ | $CH_2O$ | | |
| 133 | 3-cyclo-$C_6H_{11}$ | $CH_2O$ | | |
| 134 | 4-cyclo-$C_6H_{11}$ | $CH_2O$ | | |
| 135 | 2,4-$(cyclo-C_6H_{11})_2$, 6-$CH_3$ | $CH_2O$ | | |
| 136 | 2-$CH_3$, 4-cyclo-$C_6H_{11}$ | $CH_2O$ | | |
| 137 | 2-$CH_3$, 4-(1,1,3,3-Tetramethylbutyl) | $CH_2O$ | | |
| 138 | 2-$CH_2C_6H_5$ | $CH_2O$ | | |
| 139 | 3-$CH_2C_6H_5$ | $CH_2O$ | | |
| 140 | 4-$CH_2C_6H_5$ | $CH_2O$ | | |
| 141 | 2-$CH_2C_6H_5$, 4-$CH_3$ | $CH_2O$ | | |
| 142 | 2-$CH_3$, 4-$CH_2C_6H_5$ | $CH_2O$ | | |
| 143 | 2-$C_6H_5$ | $CH_2O$ | | |
| 144 | 3-$C_6H_5$ | $CH_2O$ | | |
| 145 | 4-$C_6H_5$ | $CH_2O$ | | |

| Nr. | $R_m$ | X | Fp(°C) | IR($cm^{-1}$) |
|---|---|---|---|---|
| 146 | 4-(2-i-$C_3H_7$-$C_6H_4$) | $CH_2O$ | | |
| 147 | 4-$C_6H_5$, 2,6-$(CH_3)_2$ | $CH_2O$ | | |
| 148 | 2-Cl, 4-$C_6H_5$ | $CH_2O$ | | |
| 149 | 2-Br, 4-$C_6H_5$ | $CH_2O$ | | |
| 150 | 2-$C_6H_5$, 4-Cl | $CH_2O$ | | |
| 151 | 2-$C_6H_5$, 4-Br | $CH_2O$ | | |
| 152 | 2-$CH_2C_6H_5$, 4-Cl | $CH_2O$ | | |
| 153 | 2-$CH_2C_6H_5$, 4-Br | $CH_2O$ | | |
| 154 | 2-Cl, 4-$CH_2C_6H_5$ | $CH_2O$ | | |
| 155 | 2-Br, 4-$CH_2C_6H_5$ | $CH_2O$ | | |
| 156 | 2-cyclo-$C_6H_{11}$, 4-Cl | $CH_2O$ | | |
| 157 | 2-cyclo-$C_6H_{11}$, 4-Br | $CH_2O$ | | |
| 158 | 2-Cl, 4-cyclo-$C_6H_{11}$ | $CH_2O$ | | |
| 159 | 2-Br, 4-cyclo-$C_6H_{11}$ | $CH_2O$ | | |
| 160 | 2-$OCH_3$ | $CH_2O$ | | |
| 161 | 3-$OCH_3$ | $CH_2O$ | | |
| 162 | 4-$OCH_3$ | $CH_2O$ | | |
| 163 | 2,4-$(OCH_3)_2$ | $CH_2O$ | | |
| 164 | 2-$OC_2H_5$ | $CH_2O$ | | |
| 165 | 3-$OC_2H_5$ | $CH_2O$ | | |
| 166 | 4-$OC_2H_5$ | $CH_2O$ | | |
| 167 | 2-$OCH_2C_6H_5$ | $CH_2O$ | | |
| 168 | 3-$OCH_2C_6H_5$ | $CH_2O$ | | |
| 169 | 4-$OCH_2C_6H_5$ | $CH_2O$ | | |
| 170 | 2-O-t-$C_4H_9$ | $CH_2O$ | | |
| 171 | 3-O-t-$C_4H_9$ | $CH_2O$ | | |

| Nr. | $R_m$ | X | Fp(°C) | IR($cm^{-1}$) |
|---|---|---|---|---|
| 172 | 4-O-t-$C_4H_9$ | $CH_2O$ | | |
| 173 | 2-$OC_6H_5$ | $CH_2O$ | | |
| 174 | 3-$OC_6H_5$ | $CH_2O$ | | |
| 175 | 4-$OC_6H_5$ | $CH_2O$ | | |
| 176 | 2-$CF_3$ | $CH_2O$ | | |
| 177 | 3-$CF_3$ | $CH_2O$ | | |
| 178 | 4-$CF_3$ | $CH_2O$ | | |
| 179 | 2-$OCF_3$ | $CH_2O$ | | |
| 180 | 3-$OCF_3$ | $CH_2O$ | | |
| 181 | 4-$OCF_3$ | $CH_2O$ | | |
| 182 | 3-$OCH_2CHF_2$ | $CH_2O$ | | |
| 183 | 3-$OCF_2CHF_2$ | $CH_2O$ | | |
| 184 | 3-$OC_2F_5$ | $CH_2O$ | | |
| 185 | 2-$NO_2$ | $CH_2O$ | | |
| 186 | 3-$NO_2$ | $CH_2O$ | | |
| 187 | 4-$NO_2$ | $CH_2O$ | | |
| 188 | 2-CN | $CH_2O$ | | |
| 189 | 3-CN | $CH_2O$ | | |
| 190 | 4-CN | $CH_2O$ | | |
| 191 | 2-$CH_3$, 3-Cl | $CH_2O$ | | |
| 192 | 2-$CH_3$, 4-Cl | $CH_2O$ | | |
| 193 | 2-$CH_3$, 5-Cl | $CH_2O$ | | |
| 194 | 2-$CH_3$, 6-Cl | $CH_2O$ | | |
| 195 | 2-$CH_3$, 3-F | $CH_2O$ | | |
| 196 | 2-$CH_3$, 4-F | $CH_2O$ | | |
| 197 | 2-$CH_3$, 5-F | $CH_2O$ | | |

| Nr. | $R_m$ | X | Fp(°C) | IR($cm^{-1}$) |
|---|---|---|---|---|
| 198 | 2-$CH_3$, 6-F | $CH_2O$ | | |
| 199 | 2-$CH_3$, 3-Br | $CH_2O$ | | |
| 200 | 2-$CH_3$, 4-Br | $CH_2O$ | | |
| 201 | 2-$CH_3$, 5-Br | $CH_2O$ | | |
| 202 | 2-$CH_3$, 6-Br | $CH_2O$ | | |
| 203 | 2-Cl, 3-$CH_3$ | $CH_2O$ | | |
| 204 | 2-Cl, 4-$CH_3$ | $CH_2O$ | | |
| 205 | 2-Cl, 5-$CH_3$ | $CH_2O$ | | |
| 206 | 2-F, 3-$CH_3$ | $CH_2O$ | | |
| 207 | 2-F, 4-$CH_3$ | $CH_2O$ | | |
| 208 | 2-F, 5-$CH_3$ | $CH_2O$ | | |
| 209 | 2-Br, 3-$CH_3$ | $CH_2O$ | | |
| 210 | 2-Br, 4-$CH_3$ | $CH_2O$ | | |
| 211 | 2-Br, 5-$CH_3$ | $CH_2O$ | | |
| 212 | 3-$CH_3$, 4-Cl | $CH_2O$ | | |
| 213 | 3-$CH_3$, 5-Cl | $CH_2O$ | | |
| 214 | 3-$CH_3$, 4-F | $CH_2O$ | | |
| 215 | 3-$CH_3$, 5-F | $CH_2O$ | | |
| 216 | 3-$CH_3$, 4-Br | $CH_2O$ | | |
| 217 | 3-$CH_3$, 5-Br | $CH_2O$ | | |
| 218 | 3-F, 4-$CH_3$ | $CH_2O$ | | |
| 219 | 3-Cl, 4-$CH_3$ | $CH_2O$ | | |
| 220 | 3-Br, 4-$CH_3$ | $CH_2O$ | | |
| 221 | 2-Cl, 4,5-$(CH_3)_2$ | $CH_2O$ | | |
| 222 | 2-Br, 4,5-$(CH_3)_2$ | $CH_2O$ | | |
| 223 | 2-Cl, 3,5-$(CH_3)_2$ | $CH_2O$ | | |

| Nr. | $R_m$ | X | Fp(°C) | IR($cm^{-1}$) |
|---|---|---|---|---|
| 224 | 2-Br, 3,5-$(CH_3)_2$ | $CH_2O$ | | |
| 225 | 2,6-$Cl_2$, 4-$CH_3$ | $CH_2O$ | | |
| 226 | 2,6-$F_2$, 4-$CH_3$ | $CH_2O$ | | |
| 227 | 2,6-$Br_2$, 4-$CH_3$ | $CH_2O$ | | |
| 228 | 2,4-$Cl_2$, 6-$CH_3$ | $CH_2O$ | | |
| 229 | 2,4-$F_2$, 6-$CH_3$ | $CH_2O$ | | |
| 230 | 2,4-$Br_2$, 6-$CH_3$ | $CH_2O$ | | |
| 231 | 2,6-$(CH_3)_2$, 4-F | $CH_2O$ | | |
| 232 | 2,6-$(CH_3)_2$, 4-Cl | $CH_2O$ | | |
| 233 | 2,6-$(CH_3)_2$, 4-Br | $CH_2O$ | | |
| 234 | 3,5-$(CH_3)_2$, 4-F | $CH_2O$ | | |
| 235 | 3,5-$(CH_3)_2$, 4-Cl | $CH_2O$ | | |
| 236 | 3,5-$(CH_3)_2$, 4-Br | $CH_2O$ | | |
| 237 | 2,3,6-$(CH_3)_3$, 4-F | $CH_2O$ | | |
| 238 | 2,3,6-$(CH_3)_3$, 4-Cl | $CH_2O$ | | |
| 239 | 2,3,6-$(CH_3)_3$, 4-Br | $CH_2O$ | | |
| 240 | 2,4-$(CH_3)_2$, 6-F | $CH_2O$ | | |
| 241 | 2,4-$(CH_3)_2$, 6-Cl | $CH_2O$ | | |
| 242 | 2,4-$(CH_3)_2$, 6-Br | $CH_2O$ | | |
| 243 | 2-i-$C_3H_7$, 4-Cl, 5-$CH_3$ | $CH_2O$ | | |
| 244 | 2-$CH_3$, 4-Cl, 5-i-$C_3H_7$ | $CH_2O$ | | |
| 245 | 2-Cl, 3-i-$C_3H_7$ | $CH_2O$ | | |
| 246 | 2-Cl, 4-i-$C_3H_7$ | $CH_2O$ | | |
| 247 | 2-Cl, 4-$NO_2$ | $CH_2O$ | | |
| 248 | 2-$NO_2$, 4-Cl | $CH_2O$ | | |

| Nr. | $R_m$ | X | Fp(°C) | IR($cm^{-1}$) |
|---|---|---|---|---|
| 249 | 2-$OCH_3$, 5-$NO_2$ | $CH_2O$ | | |
| 250 | 2,4-$Cl_2$, 5-$NO_2$ | $CH_2O$ | | |
| 251 | 2,4-$Cl_2$, 6-$NO_2$ | $CH_2O$ | | |
| 252 | 2,6-$Cl_2$, 4-$NO_2$ | $CH_2O$ | | |
| 253 | 2,6-$Br_2$, 4-$NO_2$ | $CH_2O$ | | |
| 254 | 2,6-$J_2$, 4-$NO_2$ | $CH_2O$ | | |
| 255 | H | $OCH_2$ | | |
| 256 | 2-F | $OCH_2$ | | |
| 257 | 3-F | $OCH_2$ | | |
| 258 | 4-F | $OCH_2$ | | |
| 259 | 2,3-$F_2$ | $OCH_2$ | | |
| 260 | 2,4-$F_2$ | $OCH_2$ | | |
| 261 | 2,4,6-$F_3$ | $OCH_2$ | | |
| 262 | 2,3,4,5,6-$F_5$ | $OCH_2$ | | |
| 263 | 2-Cl | $OCH_2$ | | |
| 264 | 3-Cl | $OCH_2$ | | |
| 265 | 4-Cl | $OCH_2$ | | |
| 266 | 2,3-$Cl_2$ | $OCH_2$ | | |
| 267 | 2,4-$Cl_2$ | $OCH_2$ | | |
| 268 | 2,5-$Cl_2$ | $OCH_2$ | | |
| 269 | 2,6-$Cl_2$ | $OCH_2$ | | |

| Nr. | $R_m$ | X | Fp($^oC$) | IR($cm^{-1}$) |
|---|---|---|---|---|
| 270 | 3,4-$Cl_2$ | $OCH_2$ | | |
| 271 | 3,5-$Cl_2$ | $OCH_2$ | | |
| 272 | 2,3,4-$Cl_3$ | $OCH_2$ | | |
| 273 | 2,3,5-$Cl_3$ | $OCH_2$ | | |
| 274 | 2,3,6-$Cl_3$ | $OCH_2$ | | |
| 275 | 2,4,5-$Cl_3$ | $OCH_2$ | | |
| 276 | 2,4,6-$Cl_3$ | $OCH_2$ | | |
| 277 | 3,4,5-$Cl_3$ | $OCH_2$ | | |
| 278 | 2,3,4,6-$Cl_4$ | $OCH_2$ | | |
| 279 | 2,3,5,6-$Cl_4$ | $OCH_2$ | | |
| 280 | 2,3,4,5,6-$Cl_5$ | $OCH_2$ | | |
| 281 | 2-Br | $OCH_2$ | | |
| 282 | 3-Br | $OCH_2$ | | |
| 283 | 4-Br | $OCH_2$ | | |
| 284 | 2,4-$Br_2$ | $OCH_2$ | | |
| 285 | 2,5-$Br_2$ | $OCH_2$ | | |
| 286 | 2,6-$Br_2$ | $OCH_2$ | | |
| 287 | 2,4,6-$Br_3$ | $OCH_2$ | | |
| 288 | 2,3,4,5,6-$Br_5$ | $OCH_2$ | | |
| 289 | 2-J | $OCH_2$ | | |
| 290 | 3-J | $OCH_2$ | | |
| 291 | 4-J | $OCH_2$ | | |
| 292 | 2,4-$J_2$ | $OCH_2$ | | |
| 293 | 2-Cl, 3-F | $OCH_2$ | | |
| 294 | 2-Cl, 4-F | $OCH_2$ | | |
| 295 | 2-Cl, 5-F | $OCH_2$ | | |

| Nr. | $R_m$ | X | Fp(°C) | IR($cm^{-1}$) |
|---|---|---|---|---|
| 296 | 2-Cl, 3-F | $OCH_2$ | | |
| 297 | 2-Cl, 3-Br | $OCH_2$ | | |
| 298 | 2-Cl, 4-Br | $OCH_2$ | | |
| 299 | 2-Cl, 5-Br | $OCH_2$ | | |
| 300 | 2-Cl, 6-Br | $OCH_2$ | | |
| 301 | 2-Br, 3-Cl | $OCH_2$ | | |
| 302 | 2-Br, 4-Cl | $OCH_2$ | | |
| 303 | 2-Br, 5-Cl | $OCH_2$ | | |
| 304 | 2-Br, 3-F | $OCH_2$ | | |
| 305 | 2-Br, 4-F | $OCH_2$ | | |
| 305 | 2-Br, 5-F | $OCH_2$ | | |
| 307 | 2-Br, 6-F | $OCH_2$ | | |
| 308 | 2-F, 3-Cl | $OCH_2$ | | |
| 309 | 2-F, 4-Cl | $OCH_2$ | | |
| 310 | 2-F, 5-Cl | $OCH_2$ | | |
| 311 | 3-Cl, 4-F | $OCH_2$ | | |
| 312 | 3-Cl, 5-F | $OCH_2$ | | |
| 313 | 3-Cl, 4-Br | $OCH_2$ | | |
| 314 | 3-Cl, 5-Br | $OCH_2$ | | |
| 315 | 3-F, 4-Cl | $OCH_2$ | | |
| 316 | 3-F, 4-Br | $OCH_2$ | | |
| 317 | 3-Br, 4-Cl | $OCH_2$ | | |
| 318 | 3-Br, 4-F | $OCH_2$ | | |
| 319 | 2,6-$Cl_2$, 4-Br | $OCH_2$ | | |
| 320 | 2-$CH_3$ | $OCH_2$ | | |
| 321 | 3-$CH_3$ | $OCH_2$ | | |

| Nr. | $R_m$ | X | Fp(°C) | IR($cm^{-1}$) |
|---|---|---|---|---|
| 322 | $4-CH_3$ | $OCH_2$ | | |
| 323 | $2,3-(CH_3)_2$ | $OCH_2$ | | |
| 324 | $2,4-(CH_3)_2$ | $OCH_2$ | | |
| 325 | $2,5-(CH_3)_2$ | $OCH_2$ | | |
| 326 | $2,6-(CH_3)_2$ | $OCH_2$ | | |
| 327 | $3,4-(CH_3)_2$ | $OCH_2$ | | |
| 328 | $3,5-(CH_3)_2$ | $OCH_2$ | | |
| 329 | $2,3,4-(CH_3)_3$ | $OCH_2$ | | |
| 330 | $2,3,5-(CH_3)_3$ | $OCH_2$ | | |
| 331 | $2,3,6-(CH_3)_3$ | $OCH_2$ | | |
| 332 | $2,4,5-(CH_3)_3$ | $OCH_2$ | | |
| 333 | $2,4,6-(CH_3)_3$ | $OCH_2$ | | |
| 334 | $3,4,5-(CH_3)_3$ | $OCH_2$ | | |
| 335 | $2,3,4,6-(CH_3)_4$ | $OCH_2$ | | |
| 336 | $2,3,5,6-(CH_3)_4$ | $OCH_2$ | | |
| 337 | $2,3,4,5,6-(CH_3)_5$ | $OCH_2$ | | |
| 338 | $2-C_2H_5$ | $OCH_2$ | | |
| 339 | $3-C_2H_5$ | $OCH_2$ | | |
| 340 | $4-C_2H_5$ | $OCH_2$ | | |
| 341 | $2,4-(C_2H_5)_2$ | $OCH_2$ | | |
| 342 | $2,6-(C_2H_5)_2$ | $OCH_2$ | | |
| 343 | $3,5-(C_2H_5)_2$ | $OCH_2$ | | |
| 344 | $2,4,6-(C_2H_5)_3$ | $OCH_2$ | | |
| 345 | $2-n-C_3H_7$ | $OCH_2$ | | |
| 346 | $3-n-C_3H_7$ | $OCH_2$ | | |
| 347 | $4-n-C_3H_7$ | $OCH_2$ | | |

| Nr. | $R_m$ | X | Fp(°C) | IR($cm^{-1}$) |
|---|---|---|---|---|
| 348 | 2-i-$C_2H_7$ | $OCH_2$ | | |
| 349 | 3-i-$C_2H_7$ | $OCH_2$ | | |
| 350 | 4-i-$C_3H_7$ | $OCH_2$ | | |
| 351 | 2,4-(i-$C_3H_7$)$_2$ | $OCH_2$ | | |
| 352 | 2,6-(i-$C_3H_7$)$_2$ | $OCH_2$ | | |
| 353 | 2,4,6-(i-$C_3H_7$)$_3$ | $OCH_2$ | | |
| 354 | 2,4,6-(i-$C_3H_7$)$_3$ | $OCH_2$ | | |
| 355 | 2-s-$C_4H_9$ | $OCH_2$ | | |
| 356 | 3-s-$C_4H_9$ | $OCH_2$ | | |
| 357 | 4-s-$C_4H_9$ | $OCH_2$ | | |
| 358 | 2-t-$C_4H_9$ | $OCH_2$ | | |
| 359 | 3-t-$C_4H_9$ | $OCH_2$ | | |
| 360 | 4-t-$C_4H_9$ | $OCH_2$ | | |
| 361 | 2,3-(t-$C_4H_9$)$_2$ | $OCH_2$ | | |
| 362 | 2,4-(t-$C_4H_9$)$_2$ | $OCH_2$ | | |
| 363 | 2,5-(t-$C_4H_9$)$_2$ | $OCH_2$ | | |
| 364 | 2,6-(t-$C_4H_9$)$_2$ | $OCH_2$ | | |
| 365 | 3,5-(t-$C_4H_9$)$_2$ | $OCH_2$ | | |
| 366 | 2,4,6-(t-$C_4H_9$)$_3$ | $OCH_2$ | | |
| 367 | 4-n-$C_9H_{19}$ | $OCH_2$ | | |
| 368 | 4-n-$C_{12}H_{25}$ | $OCH_2$ | | |
| 369 | 3-n-$C_{15}H_{31}$ | $OCH_2$ | | |
| 370 | 4-(1,1,3,3,-Tetramethylbutyl) | $OCH_2$ | | |
| 371 | 4-(1,1,3,-Trimethylbutyl) | $OCH_2$ | | |
| 372 | 2-t-$C_4H_9$, 4-$CH_3$ | $OCH_2$ | | |
| 373 | 2-t-$C_4H_9$, 5-$CH_3$ | $OCH_2$ | | |

| Nr. | $R_m$ | X | Fp(°C) | IR($cm^{-1}$) |
|---|---|---|---|---|
| 374 | 2,6-$(t\text{-}C_4H_9)_2$, 4-$CH_3$ | $OCH_2$ | | |
| 375 | 2-$CH_3$, 4-t-$C_4H_9$ | $OCH_2$ | | |
| 376 | 2-$CH_3$, 6-t-$C_4H_9$ | $OCH_2$ | | |
| 377 | 2-$CH_3$, 4-i-$C_3H_7$ | $OCH_2$ | | |
| 378 | 2-$CH_3$, 5-i-$C_3H_7$ | $OCH_2$ | | |
| 379 | 3-$CH_3$, 4-i-$C_3H_7$ | $OCH_2$ | | |
| 380 | 2-i-$C_3H_7$, 5-$CH_3$ | $OCH_2$ | | |
| 381 | 2,4-$(t\text{-}C_4H_9)_2$, 6-i-$C_3H_7$ | $OCH_2$ | | |
| 382 | 2-$C_3H_5$ (=Allyl) | $OCH_2$ | | |
| 383 | 3-$C_3H_5$ | $OCH_2$ | | |
| 384 | 4-$C_3H_5$ | $OCH_2$ | | |
| 385 | 2-$C_3H_5$, 6-$CH_3$ | $OCH_2$ | | |
| 386 | 2-cyclo-$C_6H_{11}$ | $OCH_2$ | | |
| 387 | 3-cyclo-$C_6H_{11}$ | $OCH_2$ | | |
| 388 | 4-cyclo-$C_6H_{11}$ | $OCH_2$ | | |
| 389 | 2,4-$(cyclo\text{-}C_6H_{11})_2$, 6-$CH_3$ | $OCH_2$ | | |
| 390 | 2-$CH_3$, 4-cyclo-$C_6H_{11}$ | $OCH_2$ | | |
| 391 | 2-$CH_3$, 4-(1,1,3,3-Tetramethylbutyl) | $OCH_2$ | | |
| 392 | 2-$CH_2C_6H_5$ | $OCH_2$ | | |
| 393 | 3-$CH_2C_6H_5$ | $OCH_2$ | | |
| 394 | 4-$CH_2C_6H_5$ | $OCH_2$ | | |
| 395 | 2-$CH_2C_6H_5$, 4-$CH_3$ | $OCH_2$ | | |
| 396 | 2-$CH_3$, 4-$CH_2C_6H_5$ | $OCH_2$ | | |
| 397 | 2-$C_6H_5$ | $OCH_2$ | | |
| 398 | 3-$C_6H_5$ | $OCH_2$ | | |
| 399 | 4-$C_6H_5$ | $OCH_2$ | | |

| Nr. | $R_m$ | X | Fp(°C) | IR($cm^{-1}$) |
|---|---|---|---|---|
| 400 | 4-(2-i-$C_3H_7$-$C_6H_4$) | $OCH_2$ | | |
| 401 | 4-$C_6H_5$, 2,6-$(CH_3)_2$ | $OCH_2$ | | |
| 402 | 2-Cl, 4-$C_6H_5$ | $OCH_2$ | | |
| 403 | 2-Br, 4-$C_6H_5$ | $OCH_2$ | | |
| 404 | 2-$C_6H_5$, 4-Cl | $OCH_2$ | | |
| 405 | 2-$C_6H_5$, 4-Br | $OCH_2$ | | |
| 406 | 2-$CH_2C_6H_5$, 4-Cl | $OCH_2$ | | |
| 407 | 2-$CH_2C_6H_5$, 4-Br | $OCH_2$ | | |
| 408 | 2-Cl, 4-$CH_2C_6H_5$ | $OCH_2$ | | |
| 409 | 2-Br, 4-$CH_2C_6H_5$ | $OCH_2$ | | |
| 410 | 2-cyclo-$C_6H_{11}$, 4-Cl | $OCH_2$ | | |
| 411 | 2-cyclo-$C_6H_{11}$, 4-Br | $OCH_2$ | | |
| 412 | 2-Cl, 4-cyclo-$C_6H_{11}$ | $OCH_2$ | | |
| 413 | 2-Br, 4-cyclo-$C_6H_{11}$ | $OCH_2$ | | |
| 414 | 2-$OCH_3$ | $OCH_2$ | | |
| 415 | 3-$OCH_3$ | $OCH_2$ | | |
| 416 | 4-$OCH_3$ | $OCH_2$ | | |
| 417 | 2,4-$(OCH_3)_2$ | $OCH_2$ | | |
| 418 | 2-$OC_2H_5$ | $OCH_2$ | | |
| 419 | 3-$OC_2H_5$ | $OCH_2$ | | |
| 420 | 4-$OC_2H_5$ | $OCH_2$ | | |
| 421 | 2-$OCH_2C_6H_5$ | $OCH_2$ | | |
| 422 | 3-$OCH_2C_6H_5$ | $OCH_2$ | | |
| 423 | 4-$OCH_2C_6H_5$ | $OCH_2$ | | |
| 424 | 2-O-t-$C_4H_9$ | $OCH_2$ | | |
| 425 | 3-O-t-$C_4H_9$ | $OCH_2$ | | |

| Nr. | $R_m$ | X | Fp(°C) | IR($cm^{-1}$) |
|---|---|---|---|---|
| 426 | 4-O-t-$C_4H_9$ | $OCH_2$ | | |
| 427 | 2-$OC_6H_5$ | $OCH_2$ | | |
| 428 | 3-$OC_6H_5$ | $OCH_2$ | | |
| 429 | 4-$OC_6H_5$ | $OCH_2$ | | |
| 430 | 2-$CF_3$ | $OCH_2$ | | |
| 431 | 3-$CF_3$ | $OCH_2$ | | |
| 432 | 4-$CF_3$ | $OCH_2$ | | |
| 433 | 2-$OCF_3$ | $OCH_2$ | | |
| 434 | 3-$OCF_3$ | $OCH_2$ | | |
| 435 | 4-$OCF_3$ | $OCH_2$ | | |
| 436 | 3-$OCH_2CHF_2$ | $OCH_2$ | | |
| 437 | 3-$OCF_2CHF_2$ | $OCH_2$ | | |
| 438 | 3-$OC_2F_5$ | $OCH_2$ | | |
| 439 | 2-$NO_2$ | $OCH_2$ | | |
| 440 | 3-$NO_2$ | $OCH_2$ | | |
| 441 | 4-$NO_2$ | $OCH_2$ | | |
| 442 | 2-CN | $OCH_2$ | | |
| 443 | 3-CN | $OCH_2$ | | |
| 444 | 4-CN | $OCH_2$ | | |
| 445 | 2-$CH_3$, 3-Cl | $OCH_2$ | | |
| 446 | 2-$CH_3$, 4-Cl | $OCH_2$ | | |
| 447 | 2-$CH_3$, 5-Cl | $OCH_2$ | | |
| 448 | 2-$CH_3$, 6-Cl | $OCH_2$ | | |
| 449 | 2-$CH_3$, 3-F | $OCH_2$ | | |
| 450 | 2-$CH_3$, 4-F | $OCH_2$ | | |
| 451 | 2-$CH_3$, 5-F | $OCH_2$ | | |

| Nr. | $R_m$ | X | Fp(°C) | IR($cm^{-1}$) |
|---|---|---|---|---|
| 452 | 2-$CH_3$, 6-F | $OCH_2$ | | |
| 453 | 2-$CH_3$, 3-Br | $OCH_2$ | | |
| 454 | 2-$CH_3$, 4-Br | $OCH_2$ | | |
| 455 | 2-$CH_3$, 5-Br | $OCH_2$ | | |
| 456 | 2-$CH_3$, 6-Br | $OCH_2$ | | |
| 457 | 2-Cl, 3-$CH_3$ | $OCH_2$ | | |
| 458 | 2-Cl, 4-$CH_3$ | $OCH_2$ | | |
| 459 | 2-Cl, 5-$CH_3$ | $OCH_2$ | | |
| 460 | 2-F, 3-$CH_3$ | $OCH_2$ | | |
| 461 | 2-F, 4-$CH_3$ | $OCH_2$ | | |
| 462 | 2-F, 5-$CH_3$ | $OCH_2$ | | |
| 463 | 2-Br, 3-$CH_3$ | $OCH_2$ | | |
| 464 | 2-Br, 4-$CH_3$ | $OCH_2$ | | |
| 465 | 2-Br, 5-$CH_3$ | $OCH_2$ | | |
| 466 | 3-$CH_3$, 4-Cl | $OCH_2$ | | |
| 467 | 3-$CH_3$, 5-Cl | $OCH_2$ | | |
| 468 | 3-$CH_3$, 4-F | $OCH_2$ | | |
| 469 | 3-$CH_3$, 5-F | $OCH_2$ | | |
| 470 | 3-$CH_3$, 4-Br | $OCH_2$ | | |
| 471 | 3-$CH_3$, 5-Br | $OCH_2$ | | |
| 472 | 3-F, 4-$CH_3$ | $OCH_2$ | | |
| 473 | 3-Cl, 4-$CH_3$ | $OCH_2$ | | |
| 474 | 3-Br, 4-$CH_3$ | $OCH_2$ | | |
| 475 | 2-Cl, 4,5-$(CH_3)_2$ | $OCH_2$ | | |
| 476 | 2-Br, 4,5-$(CH_3)_2$ | $OCH_2$ | | |
| 477 | 2-Cl, 3,5-$(CH_3)_2$ | $OCH_2$ | | |

| Nr. | $R_m$ | X | Fp(°C) | IR($cm^{-1}$) |
|---|---|---|---|---|
| 478 | 2-Br, 3,5-$(CH_3)_2$ | $OCH_2$ | | |
| 479 | 2,6-$Cl_2$, 4-$CH_3$ | $OCH_2$ | | |
| 480 | 2,6-$F_2$, 4-$CH_3$ | $OCH_2$ | | |
| 481 | 2,6-$Br_2$, 4-$CH_3$ | $OCH_2$ | | |
| 482 | 2,4-$Cl_2$, 6-$CH_3$ | $OCH_2$ | | |
| 483 | 2,4-$F_2$, 6-$CH_3$ | $OCH_2$ | | |
| 484 | 2,4-$Br_2$, 6-$CH_3$ | $OCH_2$ | | |
| 485 | 2,6-$(CH_3)_2$, 4-F | $OCH_2$ | | |
| 486 | 2,6-$(CH_3)_2$, 4-Cl | $OCH_2$ | | |
| 487 | 2,6-$(CH_3)_2$, 4-Br | $OCH_2$ | | |
| 488 | 3,5-$(CH_3)_2$, 4-F | $OCH_2$ | | |
| 489 | 3,5-$(CH_3)_2$, 4-Cl | $OCH_2$ | | |
| 490 | 3,5-$(CH_3)_2$, 4-Br | $OCH_2$ | | |
| 491 | 2,3,6-$(CH_3)_3$, 4-F | $OCH_2$ | | |
| 492 | 2,3,6-$(CH_3)_3$, 4-Cl | $OCH_2$ | | |
| 493 | 2,3,6-$(CH_3)_3$, 4-Br | $OCH_2$ | | |
| 494 | 2,4-$(CH_3)_2$, 6-F | $OCH_2$ | | |
| 495 | 2,4-$(CH_3)_2$, 6-Cl | $OCH_2$ | | |
| 496 | 2,4-$(CH_3)_2$, 6-Br | $OCH_2$ | | |
| 497 | 2-i-$C_3H_7$, 4-Cl, 5-$CH_3$ | $OCH_2$ | | |
| 498 | 2-$CH_3$, 4-Cl, 5-i-$C_3H_7$ | $OCH_2$ | | |
| 499 | 2-Cl, 3-i-$C_3H_7$ | $OCH_2$ | | |
| 500 | 2-Cl, 4-i-$C_3H_7$ | $OCH_2$ | | |
| 501 | 2-Cl, 4-$NO_2$ | $OCH_2$ | | |
| 502 | 2-$NO_2$, 4-Cl | $OCH_2$ | | |

| Nr. | $R_m$ | X | Fp(°C) | IR($cm^{-1}$) |
|---|---|---|---|---|
| 503 | 2-$OCH_3$, 5-$NO_2$ | $OCH_2$ | | |
| 504 | 2,4-$Cl_2$, 5-$NO_2$ | $OCH_2$ | | |
| 505 | 2,4-$Cl_2$, 6-$NO_2$ | $OCH_2$ | | |
| 506 | 2,6-$Cl_2$, 4-$NO_2$ | $OCH_2$ | | |
| 507 | 2,6-$Br_2$, 4-$NO_2$ | $OCH_2$ | | |
| 508 | 2,6-$J_2$, 4-$NO_2$ | $OCH_2$ | | |
| 509 | H | $CH_2$-$CH_2$ | | |
| 510 | 2-F | $CH_2$-$CH_2$ | | |
| 511 | 3-F | $CH_2$-$CH_2$ | | |
| 512 | 4-F | $CH_2$-$CH_2$ | | |
| 513 | 2,3-$F_2$ | $CH_2$-$CH_2$ | | |
| 514 | 2,4-$F_2$ | $CH_2$-$CH_2$ | | |
| 515 | 2,4,6-$F_3$ | $CH_2$-$CH_2$ | | |
| 516 | 2,3,4,5,6-$F_5$ | $CH_2$-$CH_2$ | | |
| 517 | 2-Cl | $CH_2$-$CH_2$ | | |
| 518 | 3-Cl | $CH_2$-$CH_2$ | | |
| 519 | 4-Cl | $CH_2$-$CH_2$ | | |
| 520 | 2,3-$Cl_2$ | $CH_2$-$CH_2$ | | |
| 521 | 2,4-$Cl_2$ | $CH_2$-$CH_2$ | | |
| 522 | 2,5-$Cl_2$ | $CH_2$-$CH_2$ | | |
| 523 | 2,6-$Cl_2$ | $CH_2$-$CH_2$ | | |

| Nr. | $R_m$ | X | Fp(°C) | IR($cm^{-1}$) |
|---|---|---|---|---|
| 524 | 3,4-$Cl_2$ | $CH_2$-$CH_2$ | | |
| 525 | 3,5-$Cl_2$ | $CH_2$-$CH_2$ | | |
| 526 | 2,3,4-$Cl_3$ | $CH_2$-$CH_2$ | | |
| 527 | 2,3,5-$Cl_3$ | $CH_2$-$CH_2$ | | |
| 528 | 2,3,6-$Cl_3$ | $CH_2$-$CH_2$ | | |
| 529 | 2,4,5-$Cl_3$ | $CH_2$-$CH_2$ | | |
| 530 | 2,4,6-$Cl_3$ | $CH_2$-$CH_2$ | | |
| 531 | 3,4,5-$Cl_3$ | $CH_2$-$CH_2$ | | |
| 532 | 2,3,4,6-$Cl_4$ | $CH_2$-$CH_2$ | | |
| 533 | 2,3,5,6-$Cl_4$ | $CH_2$-$CH_2$ | | |
| 534 | 2,3,4,5,6-$Cl_5$ | $CH_2$-$CH_2$ | | |
| 535 | 2-Br | $CH_2$-$CH_2$ | | |
| 536 | 3-Br | $CH_2$-$CH_2$ | | |
| 537 | 4-Br | $CH_2$-$CH_2$ | | |
| 538 | 2,4-$Br_2$ | $CH_2$-$CH_2$ | | |
| 539 | 2,5-$Br_2$ | $CH_2$-$CH_2$ | | |
| 540 | 2,6-$Br_2$ | $CH_2$-$CH_2$ | | |
| 541 | 2,4,6-$Br_3$ | $CH_2$-$CH_2$ | | |
| 542 | 2,3,4,5,6-$Br_5$ | $CH_2$-$CH_2$ | | |
| 543 | 2-J | $CH_2$-$CH_2$ | | |
| 544 | 3-J | $CH_2$-$CH_2$ | | |
| 545 | 4-J | $CH_2$-$CH_2$ | | |
| 546 | 2,4-$J_2$ | $CH_2$-$CH_2$ | | |
| 547 | 2-Cl, 3-F | $CH_2$-$CH_2$ | | |
| 548 | 2-Cl, 4-F | $CH_2$-$CH_2$ | | |
| 549 | 2-Cl, 5-F | $CH_2$-$CH_2$ | | |

| Nr. | $R_m$ | X | Fp(°C) | IR($cm^{-1}$) |
|---|---|---|---|---|
| 550 | 2-Cl, 6-F | $CH_2-CH_2$ | | |
| 551 | 2-Cl, 3-Br | $CH_2-CH_2$ | | |
| 552 | 2-Cl, 4-Br | $CH_2-CH_2$ | | |
| 553 | 2-Cl, 5-Br | $CH_2-CH_2$ | | |
| 554 | 2-Cl, 6-Br | $CH_2-CH_2$ | | |
| 555 | 2-Br, 3-Cl | $CH_2-CH_2$ | | |
| 556 | 2-Br, 4-Cl | $CH_2-CH_2$ | | |
| 357 | 2-Br, 5-Cl | $CH_2-CH_2$ | | |
| 558 | 2-Br, 3-F | $CH_2-CH_2$ | | |
| 559 | 2-Br, 4-F | $CH_2-CH_2$ | | |
| 560 | 2-Br, 5-F | $CH_2-CH_2$ | | |
| 561 | 2-Br, 6-F | $CH_2-CH_2$ | | |
| 562 | 2-F, 3-Cl | $CH_2-CH_2$ | | |
| 563 | 2-F, 4-Cl | $CH_2-CH_2$ | | |
| 564 | 2-F, 5-Cl | $CH_2-CH_2$ | | |
| 565 | 3-Cl, 4-F | $CH_2-CH_2$ | | |
| 566 | 3-Cl, 5-F | $CH_2-CH_2$ | | |
| 567 | 3-Cl, 4-Br | $CH_2-CH_2$ | | |
| 568 | 3-Cl, 5-Br | $CH_2-CH_2$ | | |
| 569 | 3-F, 4-Cl | $CH_2-CH_2$ | | |
| 570 | 3-F, 4-Br | $CH_2-CH_2$ | | |
| 571 | 3-Br, 4-Cl | $CH_2-CH_2$ | | |
| 572 | 3-Br, 4-F | $CH_2-CH_2$ | | |
| 573 | 2,6-$Cl_2$, 4-Br | $CH_2-CH_2$ | | |
| 574 | 2-$CH_3$ | $CH_2-CH_2$ | | |
| 575 | 3-$CH_3$ | $CH_2-CH_2$ | | |

| Nr. | $R_m$ | X | Fp(°C) | IR($cm^{-1}$) |
|---|---|---|---|---|
| 576 | $4-CH_3$ | $CH_2-CH_2$ | | |
| 577 | $2,3-(CH_3)_2$ | $CH_2-CH_2$ | | |
| 578 | $2,4-(CH_3)_2$ | $CH_2-CH_2$ | | |
| 579 | $2,5-(CH_3)_2$ | $CH_2-CH_2$ | | |
| 580 | $2,6-(CH_3)_2$ | $CH_2-CH_2$ | | |
| 581 | $3,4-(CH_3)_2$ | $CH_2-CH_2$ | | |
| 582 | $3,5-(CH_3)_2$ | $CH_2-CH_2$ | | |
| 583 | $2,3,4-(CH_3)_3$ | $CH_2-CH_2$ | | |
| 584 | $2,3,5-(CH_3)_3$ | $CH_2-CH_2$ | | |
| 585 | $2,3,6-(CH_3)_3$ | $CH_2-CH_2$ | | |
| 586 | $2,4,5-(CH_3)_3$ | $CH_2-CH_2$ | | |
| 587 | $2,4,6-(CH_3)_3$ | $CH_2-CH_2$ | | |
| 588 | $3,4,5-(CH_3)_3$ | $CH_2-CH_2$ | | |
| 589 | $2,3,4,6-(CH_3)_4$ | $CH_2-CH_2$ | | |
| 590 | $2,3,5,6-(CH_3)_4$ | $CH_2-CH_2$ | | |
| 591 | $2,3,4,5,6-(CH_3)_5$ | $CH_2-CH_2$ | | |
| 592 | $2-C_2H_5$ | $CH_2-CH_2$ | | |
| 593 | $3-C_2H_5$ | $CH_2-CH_2$ | | |
| 594 | $4-C_2H_5$ | $CH_2-CH_2$ | | |
| 595 | $2,4-(C_2H_5)_2$ | $CH_2-CH_2$ | | |
| 596 | $2,6-(C_2H_5)_2$ | $CH_2-CH_2$ | | |
| 597 | $3,5-(C_2H_5)_2$ | $CH_2-CH_2$ | | |
| 598 | $2,4,6-(C_2H_5)_3$ | $CH_2-CH_2$ | | |
| 599 | $2-n-C_3H_7$ | $CH_2-CH_2$ | | |
| 600 | $3-n-C_3H_7$ | $CH_2-CH_2$ | | |
| 601 | $4-n-C_3H_7$ | $CH_2-CH_2$ | | |

| Nr. | $R_m$ | X | Fp(°C) | IR($cm^{-1}$) |
|---|---|---|---|---|
| 602 | 2-i-$C_2H_7$ | $CH_2$-$CH_2$ | | |
| 603 | 3-i-$C_2H_7$ | $CH_2$-$CH_2$ | | |
| 604 | 4-i-$C_3H_7$ | $CH_2$-$CH_2$ | | |
| 605 | 2,4-(i-$C_3H_7$)$_2$ | $CH_2$-$CH_2$ | | |
| 606 | 2,6-(i-$C_3H_7$)$_2$ | $CH_2$-$CH_2$ | | |
| 607 | 2,4,6-(i-$C_3H_7$)$_3$ | $CH_2$-$CH_2$ | | |
| 608 | 2,4,6-(i-$C_3H_7$)$_3$ | $CH_2$-$CH_2$ | | |
| 609 | 2-s-$C_4H_9$ | $CH_2$-$CH_2$ | | |
| 610 | 3-s-$C_4H_9$ | $CH_2$-$CH_2$ | | |
| 611 | 4-s-$C_4H_9$ | $CH_2$-$CH_2$ | | |
| 612 | 2-t-$C_4H_9$ | $CH_2$-$CH_2$ | | |
| 613 | 3-t-$C_4H_9$ | $CH_2$-$CH_2$ | | |
| 614 | 4-t-$C_4H_9$ | $CH_2$-$CH_2$ | | |
| 615 | 2,3-(t-$C_4H_9$)$_2$ | $CH_2$-$CH_2$ | | |
| 616 | 2,4-(t-$C_4H_9$)$_2$ | $CH_2$-$CH_2$ | | |
| 617 | 2,5-(t-$C_4H_9$)$_2$ | $CH_2$-$CH_2$ | | |
| 618 | 2,6-(t-$C_4H_9$)$_2$ | $CH_2$-$CH_2$ | | |
| 619 | 3,5-(t-$C_4H_9$)$_2$ | $CH_2$-$CH_2$ | | |
| 620 | 2,4,6-(t-$C_4H_9$)$_3$ | $CH_2$-$CH_2$ | | |
| 621 | 4-n-$C_9H_{19}$ | $CH_2$-$CH_2$ | | |
| 622 | 4-n-$C_{12}H_{25}$ | $CH_2$-$CH_2$ | | |
| 623 | 3-n-$C_{15}H_{31}$ | $CH_2$-$CH_2$ | | |
| 624 | 4-(1,1,3,3,-Tetramethylbutyl) | $CH_2$-$CH_2$ | | |
| 625 | 4-(1,1,3,-Trimethylbutyl) | $CH_2$-$CH_2$ | | |
| 626 | 2-t-$C_4H_9$, 4-$CH_3$ | $CH_2$-$CH_2$ | | |
| 627 | 2-t-$C_4H_9$, 5-$CH_3$ | $CH_2$-$CH_2$ | | |

| Nr. | $R_m$ | X | Fp(°C) | IR($cm^{-1}$) |
|---|---|---|---|---|
| 628 | 2,6-(t-$C_4H_9$)$_2$, 4-$CH_3$ | $CH_2CH_2$ | | |
| 629 | 2-$CH_3$, 4-t-$C_4H_9$ | $CH_2$-$CH_2$ | | |
| 630 | 2-$CH_3$, 6-t-$C_4H_9$ | $CH_2$-$CH_2$ | | |
| 631 | 2-$CH_3$, 4-i-$C_3H_7$ | $CH_2$-$CH_2$ | | |
| 632 | 2-$CH_3$, 5-i-$C_3H_7$ | $CH_2$-$CH_2$ | | |
| 633 | 3-$CH_3$, 4-i-$C_3H_7$ | $CH_2$-$CH_2$ | | |
| 634 | 2-i-$C_3H_7$, 5-$CH_3$ | $CH_2$-$CH_2$ | | |
| 635 | 2,4-(t-$C_4H_9$)$_2$, 6-i-$C_3H_7$ | $CH_2$-$CH_2$ | | |
| 636 | 2-$C_3H_5$ (=Allyl) | $CH_2$-$CH_2$ | | |
| 637 | 3-$C_3H_5$ | $CH_2$-$CH_2$ | | |
| 638 | 4-$C_3H_5$ | $CH_2$-$CH_2$ | | |
| 639 | 2-$C_3H_5$, 6-$CH_3$ | $CH_2$-$CH_2$ | | |
| 640 | 2-cyclo-$C_6H_{11}$ | $CH_2$-$CH_2$ | | |
| 641 | 3-cyclo-$C_6H_{11}$ | $CH_2$-$CH_2$ | | |
| 642 | 4-cyclo-$C_6H_{11}$ | $CH_2$-$CH_2$ | | |
| 643 | 2,4-(cyclo-$C_6H_{11}$)$_2$, 6-$CH_3$ | $CH_2$-$CH_2$ | | |
| 644 | 2-$CH_3$, 4-cyclo-$C_6H_{11}$ | $CH_2$-$CH_2$ | | |
| 645 | 2-$CH_3$, 4-(1,1,3,3-Tetramethylbutyl) | $CH_2$-$CH_2$ | | |
| 646 | 2-$CH_2C_6H_5$ | $CH_2$-$CH_2$ | | |
| 647 | 3-$CH_2C_6H_5$ | $CH_2$-$CH_2$ | | |
| 648 | 4-$CH_2C_6H_5$ | $CH_2$-$CH_2$ | | |
| 649 | 2-$CH_2C_6H_5$, 4-$CH_3$ | $CH_2$-$CH_2$ | | |
| 650 | 2-$CH_3$, 4-$CH_2C_6H_5$ | $CH_2$-$CH_2$ | | |
| 651 | 2-$C_6H_5$ | $CH_2$-$CH_2$ | | |
| 652 | 3-$C_6H_5$ | $CH_2$-$CH_2$ | | |
| 653 | 4-$C_6H_5$ | $CH_2$-$CH_2$ | | |

| Nr. | $R_m$ | X | Fp(°C) | IR($cm^{-1}$) |
|---|---|---|---|---|
| 654 | 4-(2-i-$C_3H_7$-$C_6H_4$) | $CH_2$-$CH_2$ | | |
| 655 | 4-$C_6H_5$, 2,6-$(CH_3)_2$ | $CH_2$-$CH_2$ | | |
| 656 | 2-Cl, 4-$C_6H_5$ | $CH_2$-$CH_2$ | | |
| 657 | 2-Br, 4-$C_6H_5$ | $CH_2$-$CH_2$ | | |
| 658 | 2-$C_6H_5$, 4-Cl | $CH_2$-$CH_2$ | | |
| 659 | 2-$C_6H_5$, 4-Br | $CH_2$-$CH_2$ | | |
| 660 | 2-$CH_2C_6H_5$, 4-Cl | $CH_2$-$CH_2$ | | |
| 661 | 2-$CH_2C_6H_5$, 4-Br | $CH_2$-$CH_2$ | | |
| 662 | 2-Cl, 4-$CH_2C_6H_5$ | $CH_2$-$CH_2$ | | |
| 663 | 2-Br, 4-$CH_2C_6H_5$ | $CH_2$-$CH_2$ | | |
| 664 | 2-cyclo-$C_6H_{11}$, 4-Cl | $CH_2$-$CH_2$ | | |
| 665 | 2-cyclo-$C_6H_{11}$, 4-Br | $CH_2$-$CH_2$ | | |
| 666 | 2-Cl, 4-cyclo-$C_6H_{11}$ | $CH_2$-$CH_2$ | | |
| 667 | 2-Br, 4-cyclo-$C_6H_{11}$ | $CH_2$-$CH_2$ | | |
| 668 | 2-$OCH_3$ | $CH_2$-$CH_2$ | | |
| 669 | 3-$OCH_3$ | $CH_2$-$CH_2$ | | |
| 670 | 4-$OCH_3$ | $CH_2$-$CH_2$ | | |
| 671 | 2,4-$(OCH_3)_2$ | $CH_2$-$CH_2$ | | |
| 672 | 2-$OC_2H_5$ | $CH_2$-$CH_2$ | | |
| 673 | 3-$OC_2H_5$ | $CH_2$-$CH_2$ | | |
| 674 | 4-$OC_2H_5$ | $CH_2$-$CH_2$ | | |
| 675 | 2-$OCH_2C_6H_5$ | $CH_2$-$CH_2$ | | |
| 676 | 3-$OCH_2C_6H_5$ | $CH_2$-$CH_2$ | | |
| 677 | 4-$OCH_2C_6H_5$ | $CH_2$-$CH_2$ | | |
| 678 | 2-O-t-$C_4H_9$ | $CH_2$-$CH_2$ | | |
| 679 | 3-O-t-$C_4H_9$ | $CH_2$-$CH_2$ | | |

| Nr. | $R_m$ | X | Fp(°C) | IR($cm^{-1}$) |
|---|---|---|---|---|
| 680 | 4-O-t-$C_4H_9$ | $CH_2$-$CH_2$ | | |
| 681 | 2-$OC_6H_5$ | $CH_2$-$CH_2$ | | |
| 682 | 3-$OC_6H_5$ | $CH_2$-$CH_2$ | | |
| 683 | 4-$OC_6H_5$ | $CH_2$-$CH_2$ | | |
| 684 | 2-$CF_3$ | $CH_2$-$CH_2$ | | |
| 685 | 3-$CF_3$ | $CH_2$-$CH_2$ | | |
| 686 | 4-$CF_3$ | $CH_2$-$CH_2$ | | |
| 687 | 2-$OCF_3$ | $CH_2$-$CH_2$ | | |
| 688 | 3-$OCF_3$ | $CH_2$-$CH_2$ | | |
| 689 | 4-$OCF_3$ | $CH_2$-$CH_2$ | | |
| 690 | 3-$OCH_2CHF_2$ | $CH_2$-$CH_2$ | | |
| 691 | 3-$OCF_2CHF_2$ | $CH_2$-$CH_2$ | | |
| 692 | 3-$OC_2F_5$ | $CH_2$-$CH_2$ | | |
| 693 | 2-$NO_2$ | $CH_2$-$CH_2$ | | |
| 694 | 3-$NO_2$ | $CH_2$-$CH_2$ | | |
| 695 | 4-$NO_2$ | $CH_2$-$CH_2$ | | |
| 696 | 2-CN | $CH_2$-$CH_2$ | | |
| 697 | 3-CN | $CH_2$-$CH_2$ | | |
| 698 | 4-CN | $CH_2$-$CH_2$ | | |
| 699 | 2-$CH_3$, 3-Cl | $CH_2$-$CH_2$ | | |
| 700 | 2-$CH_3$, 4-Cl | $CH_2$-$CH_2$ | | |
| 701 | 2-$CH_3$, 5-Cl | $CH_2$-$CH_2$ | | |
| 702 | 2-$CH_3$, 6-Cl | $CH_2$-$CH_2$ | | |
| 703 | 2-$CH_3$, 3-F | $CH_2$-$CH_2$ | | |
| 704 | 2-$CH_3$, 4-F | $CH_2$-$CH_2$ | | |
| 705 | 2-$CH_3$, 5-F | $CH_2$-$CH_2$ | | |

| Nr. | $R_m$ | X | Fp(°C) | IR($cm^{-1}$) |
|---|---|---|---|---|
| 706 | 2-$CH_3$, 6-F | $CH_2$-$CH_2$ | | |
| 707 | 2-$CH_3$, 3-Br | $CH_2$-$CH_2$ | | |
| 708 | 2-$CH_3$, 4-Br | $CH_2$-$CH_2$ | | |
| 709 | 2-$CH_3$, 5-Br | $CH_2$-$CH_2$ | | |
| 710 | 2-$CH_3$, 6-Br | $CH_2$-$CH_2$ | | |
| 711 | 2-Cl, 3-$CH_3$ | $CH_2$-$CH_2$ | | |
| 712 | 2-Cl, 4-$CH_3$ | $CH_2$-$CH_2$ | | |
| 713 | 2-Cl, 5-$CH_3$ | $CH_2$-$CH_2$ | | |
| 714 | 2-F, 3-$CH_3$ | $CH_2$-$CH_2$ | | |
| 715 | 2-F, 4-$CH_3$ | $CH_2$-$CH_2$ | | |
| 716 | 2-F, 5-$CH_3$ | $CH_2$-$CH_2$ | | |
| 717 | 2-Br, 3-$CH_3$ | $CH_2$-$CH_2$ | | |
| 718 | 2-Br, 4-$CH_3$ | $CH_2$-$CH_2$ | | |
| 719 | 2-Br, 5-$CH_3$ | $CH_2$-$CH_2$ | | |
| 720 | 3-$CH_3$, 4-Cl | $CH_2$-$CH_2$ | | |
| 721 | 3-$CH_3$, 5-Cl | $CH_2$-$CH_2$ | | |
| 722 | 3-$CH_3$, 4-F | $CH_2$-$CH_2$ | | |
| 723 | 3-$CH_3$, 5-F | $CH_2$-$CH_2$ | | |
| 724 | 3-$CH_3$, 4-Br | $CH_2$-$CH_2$ | | |
| 725 | 3-$CH_3$, 5-Br | $CH_2$-$CH_2$ | | |
| 726 | 3-F, 4-$CH_3$ | $CH_2$-$CH_2$ | | |
| 727 | 3-Cl, 4-$CH_3$ | $CH_2$-$CH_2$ | | |
| 728 | 3-Br, 4-$CH_3$ | $CH_2$-$CH_2$ | | |
| 729 | 2-Cl, 4,5-$(CH_3)_2$ | $CH_2$-$CH_2$ | | |
| 730 | 2-Br, 4,5-$(CH_3)_2$ | $CH_2$-$CH_2$ | | |
| 731 | 2-Cl, 3,5-$(CH_3)_2$ | $CH_2$-$CH_2$ | | |

| Nr. | $R_m$ | X | Fp(°C) | IR($cm^{-1}$) |
|---|---|---|---|---|
| 732 | 2-Br, 3,5-$(CH_3)_2$ | $CH_2-CH_2$ | | |
| 733 | 2,6-$Cl_2$, 4-$CH_3$ | $CH_2-CH_2$ | | |
| 734 | 2,6-$F_2$, 4-$CH_3$ | $CH_2-CH_2$ | | |
| 735 | 2,6-$Br_2$, 4-$CH_3$ | $CH_2-CH_2$ | | |
| 736 | 2,4-$Cl_2$, 6-$CH_3$ | $CH_2-CH_2$ | | |
| 737 | 2,4-$F_2$, 6-$CH_3$ | $CH_2-CH_2$ | | |
| 738 | 2,4-$Br_2$, 6-$CH_3$ | $CH_2-CH_2$ | | |
| 739 | 2,6-$(CH_3)_2$, 4-F | $CH_2-CH_2$ | | |
| 740 | 2,6-$(CH_3)_2$, 4-Cl | $CH_2-CH_2$ | | |
| 741 | 2,6-$(CH_3)_2$, 4-Br | $CH_2-CH_2$ | | |
| 742 | 3,5-$(CH_3)_2$, 4-F | $CH_2-CH_2$ | | |
| 743 | 3,5-$(CH_3)_2$, 4-Cl | $CH_2-CH_2$ | | |
| 744 | 3,5-$(CH_3)_2$, 4-Br | $CH_2-CH_2$ | | |
| 745 | 2,3,6-$(CH_3)_3$, 4-F | $CH_2-CH_2$ | | |
| 746 | 2,3,6-$(CH_3)_3$, 4-Cl | $CH_2-CH_2$ | | |
| 747 | 2,3,6-$(CH_3)_3$, 4-Br | $CH_2-CH_2$ | | |
| 748 | 2,4-$(CH_3)_2$, 6-F | $CH_2-CH_2$ | | |
| 749 | 2,4-$(CH_3)_2$, 6-Cl | $CH_2-CH_2$ | | |
| 750 | 2,4-$(CH_3)_2$, 6-Br | $CH_2-CH_2$ | | |
| 751 | 2-i-$C_3H_7$, 4-Cl, 5-$CH_3$ | $CH_2-CH_2$ | | |
| 752 | 2-$CH_3$, 4-Cl, 5-i-$C_3H_7$ | $CH_2-CH_2$ | | |
| 753 | 2-Cl, 3-i-$C_3H_7$ | $CH_2-CH_2$ | | |
| 754 | 2-Cl, 4-i-$C_3H_7$ | $CH_2-CH_2$ | | |
| 755 | 2-Cl, 4-$NO_2$ | $CH_2-CH_2$ | | |
| 756 | 2-$NO_2$, 4-Cl | $CH_2-CH_2$ | | |

| Nr. | $R_m$ | X | Fp(°C) | IR($cm^{-1}$) |
|---|---|---|---|---|
| 757 | 2-$OCH_3$, 5-$NO_2$ | $CH_2$-$CH_2$ | | |
| 758 | 2,4-$Cl_2$, 5-$NO_2$ | $CH_2$-$CH_2$ | | |
| 759 | 2,4-$Cl_2$, 6-$NO_2$ | $CH_2$-$CH_2$ | | |
| 760 | 2,6-$Cl_2$, 4-$NO_2$ | $CH_2$-$CH_2$ | | |
| 761 | 2,6-$Br_2$, 4-$NO_2$ | $CH_2$-$CH_2$ | | |
| 762 | 2,6-$J_2$, 4-$NO_2$ | $CH_2$-$CH_2$ | | |
| 763 | H | CH=CH | | |
| 764 | 2-F | CH=CH | | |
| 765 | 3-F | CH=CH | | |
| 766 | 4-F | CH=CH | | |
| 767 | 2,3-$F_2$ | CH=CH | | |
| 768 | 2,4-$F_2$ | CH=CH | | |
| 769 | 2,4,6-$F_3$ | CH=CH | | |
| 770 | 2,3,4,5,6-$F_5$ | CH=CH | | |
| 771 | 2-Cl | CH=CH | | |
| 772 | 3-Cl | CH=CH | | |
| 773 | 4-Cl | CH=CH | | |
| 774 | 2,3-$Cl_2$ | CH=CH | | |
| 775 | 2,4-$Cl_2$ | CH=CH | | |
| 776 | 2,5-$Cl_2$ | CH=CH | | |
| 777 | 2,6-$Cl_2$ | CH=CH | | |

| Nr. | $R_m$ | X | Fp(°C) | IR($cm^{-1}$) |
|---|---|---|---|---|
| 778 | 3,4-$Cl_2$ | CH=CH | | |
| 779 | 3,5-$Cl_2$ | CH=CH | | |
| 780 | 2,3,4-$Cl_3$ | CH=CH | | |
| 781 | 2,3,5-$Cl_3$ | CH=CH | | |
| 782 | 2,3,6-$Cl_3$ | CH=CH | | |
| 783 | 2,4,5-$Cl_3$ | CH=CH | | |
| 784 | 2,4,6-$Cl_3$ | CH=CH | | |
| 785 | 3,4,5-$Cl_3$ | CH=CH | | |
| 786 | 2,3,4,6-$Cl_4$ | CH=CH | | |
| 787 | 2,3,5,6-$Cl_4$ | CH=CH | | |
| 788 | 2,3,4,5,6-$Cl_5$ | CH=CH | | |
| 789 | 2-Br | CH=CH | | |
| 790 | 3-Br | CH=CH | | |
| 791 | 4-Br | CH=CH | | |
| 792 | 2,4-$Br_2$ | CH=CH | | |
| 793 | 2,5-$Br_2$ | CH=CH | | |
| 794 | 2,6-$Br_2$ | CH=CH | | |
| 795 | 2,4,6-$Br_3$ | CH=CH | | |
| 796 | 2,3,4,5,6-$Br_5$ | CH=CH | | |
| 797 | 2-J | CH=CH | | |
| 798 | 3-J | CH=CH | | |
| 799 | 4-J | CH=CH | | |
| 800 | 2,4-$J_2$ | CH=CH | | |
| 801 | 2-Cl, 3-F | CH=CH | | |
| 802 | 2-Cl, 4-F | CH=CH | | |
| 803 | 2-Cl, 5-F | CH=CH | | |

| Nr. | $R_m$ | X | Fp($^oC$) | IR($cm^{-1}$) |
|---|---|---|---|---|
| 804 | 2-Cl, 6-F | CH=CH | | |
| 805 | 2-Cl, 3-Br | CH=CH | | |
| 806 | 2-Cl, 4-Br | CH=CH | | |
| 807 | 2-Cl, 5-Br | CH=CH | | |
| 808 | 2-Cl, 6-Br | CH=CH | | |
| 809 | 2-Br, 3-Cl | CH=CH | | |
| 810 | 2-Br, 4-Cl | CH=CH | | |
| 811 | 2-Br, 5-Cl | CH=CH | | |
| 812 | 2-Br, 3-F | CH=CH | | |
| 813 | 2-Br, 4-F | CH=CH | | |
| 814 | 2-Br, 5-F | CH=CH | | |
| 815 | 2-Br, 6-F | CH=CH | | |
| 816 | 2-F, 3-Cl | CH=CH | | |
| 817 | 2-F, 4-Cl | CH=CH | | |
| 818 | 2-F, 5-Cl | CH=CH | | |
| 819 | 3-Cl, 4-F | CH=CH | | |
| 820 | 3-Cl, 5-F | CH=CH | | |
| 821 | 3-Cl, 4-Br | CH=CH | | |
| 822 | 3-Cl, 5-Br | CH=CH | | |
| 823 | 3-F, 4-Cl | CH=CH | | |
| 824 | 3-F, 4-Br | CH=CH | | |
| 825 | 3-Br, 4-Cl | CH=CH | | |
| 826 | 3-Br, 4-F | CH=CH | | |
| 827 | 2,6-$Cl_2$, 4-Br | CH=CH | | |
| 828 | 2-$CH_3$ | CH=CH | | |
| 829 | 3-$CH_3$ | CH=CH | | |

| Nr. | $R_m$ | X | Fp(°C) | IR($cm^{-1}$) |
|---|---|---|---|---|
| 830 | 4-$CH_3$ | CH=CH | | |
| 831 | 2,3-$(CH_3)_2$ | CH=CH | | |
| 832 | 2,4-$(CH_3)_2$ | CH=CH | | |
| 833 | 2,5-$(CH_3)_2$ | CH=CH | | |
| 834 | 2,6-$(CH_3)_2$ | CH=CH | | |
| 835 | 3,4-$(CH_3)_2$ | CH=CH | | |
| 836 | 3,5-$(CH_3)_2$ | CH=CH | | |
| 837 | 2,3,4-$(CH_3)_3$ | CH=CH | | |
| 838 | 2,3,5-$(CH_3)_3$ | CH=CH | | |
| 839 | 2,3,6-$(CH_3)_3$ | CH=CH | | |
| 840 | 2,4,5-$(CH_3)_3$ | CH=CH | | |
| 841 | 2,4,6-$(CH_3)_3$ | CH=CH | | |
| 842 | 3,4,5-$(CH_3)_3$ | CH=CH | | |
| 843 | 2,3,4,6-$(CH_3)_4$ | CH=CH | | |
| 844 | 2,3,5,6-$(CH_3)_4$ | CH=CH | | |
| 845 | 2,3,4,5,6-$(CH_3)_5$ | CH=CH | | |
| 846 | 2-$C_2H_5$ | CH=CH | | |
| 847 | 3-$C_2H_5$ | CH=CH | | |
| 848 | 4-$C_2H_5$ | CH=CH | | |
| 849 | 2,4-$(C_2H_5)_2$ | CH=CH | | |
| 850 | 2,6-$(C_2H_5)_2$ | CH=CH | | |
| 851 | 3,5-$(C_2H_5)_2$ | CH=CH | | |
| 852 | 2,4,6-$(C_2H_5)_3$ | CH=CH | | |
| 853 | 2-n-$C_3H_7$ | CH=CH | | |
| 854 | 3-n-$C_3H_7$ | CH=CH | | |
| 855 | 4-n-$C_3H_7$ | CH=CH | | |

| Nr. | $R_m$ | X | Fp(°C) | IR($cm^{-1}$) |
|---|---|---|---|---|
| 856 | 2-i-$C_2H_7$ | CH=CH | | |
| 857 | 3-i-$C_2H_7$ | CH=CH | | |
| 858 | 4-i-$C_3H_7$ | CH=CH | | |
| 859 | 2,4-(i-$C_3H_7$)$_2$ | CH=CH | | |
| 860 | 2,6-(i-$C_3H_7$)$_2$ | CH=CH | | |
| 861 | 2,4,6-(i-$C_3H_7$)$_3$ | CH=CH | | |
| 862 | 2,4,6-(i-$C_3H_7$)$_3$ | CH=CH | | |
| 863 | 2-s-$C_4H_9$ | CH=CH | | |
| 864 | 3-s-$C_4H_9$ | CH=CH | | |
| 865 | 4-s-$C_4H_9$ | CH=CH | | |
| 866 | 2-t-$C_4H_9$ | CH=CH | | |
| 867 | 3-t-$C_4H_9$ | CH=CH | | |
| 868 | 4-t-$C_4H_9$ | CH=CH | | |
| 869 | 2,3-(t-$C_4H_9$)$_2$ | CH=CH | | |
| 870 | 2,4-(t-$C_4H_9$)$_2$ | CH=CH | | |
| 871 | 2,5-(t-$C_4H_9$)$_2$ | CH=CH | | |
| 872 | 2,6-(t-$C_4H_9$)$_2$ | CH=CH | | |
| 873 | 3,5-(t-$C_4H_9$)$_2$ | CH=CH | | |
| 874 | 2,4,6-(t-$C_4H_9$)$_3$ | CH=CH | | |
| 875 | 4-n-$C_9H_{19}$ | CH=CH | | |
| 876 | 4-n-$C_{12}H_{25}$ | CH=CH | | |
| 877 | 3-n-$C_{15}H_{31}$ | CH=CH | | |
| 878 | 4-(1,1,3,3,-Tetramethylbutyl) | CH=CH | | |
| 879 | 4-(1,1,3,-Trimethylbutyl) | CH=CH | | |
| 880 | 2-t-$C_4H_9$, 4-$CH_3$ | CH=CH | | |
| 881 | 2-t-$C_4H_9$, 5-$CH_3$ | CH=CH | | |

| Nr. | $R_m$ | X | Fp(°C) | IR($cm^{-1}$) |
|---|---|---|---|---|
| 882 | 2,6-(t-$C_4H_9$)$_2$, 4-$CH_3$ | CH=CH | | |
| 883 | 2-$CH_3$, 4-t-$C_4H_9$ | CH=CH | | |
| 884 | 2-$CH_3$, 6-t-$C_4H_9$ | CH=CH | | |
| 885 | 2-$CH_3$, 4-i-$C_3H_7$ | CH=CH | | |
| 886 | 2-$CH_3$, 5-i-$C_3H_7$ | CH=CH | | |
| 887 | 3-$CH_3$, 4-i-$C_3H_7$ | CH=CH | | |
| 888 | 2-i-$C_3H_7$, 5-$CH_3$ | CH=CH | | |
| 889 | 2,4-(t-$C_4H_9$)$_2$, 6-i-$C_3H_7$ | CH=CH | | |
| 890 | 2-$C_3H_5$ (=Allyl) | CH=CH | | |
| 891 | 3-$C_3H_5$ | CH=CH | | |
| 892 | 4-$C_3H_5$ | CH=CH | | |
| 893 | 2-$C_3H_5$, 6-$CH_3$ | CH=CH | | |
| 894 | 2-cyclo-$C_6H_{11}$ | CH=CH | | |
| 895 | 3-cyclo-$C_6H_{11}$ | CH=CH | | |
| 896 | 4-cyclo-$C_6H_{11}$ | CH=CH | | |
| 897 | 2,4-(cyclo-$C_6H_{11}$)$_2$, 6-$CH_3$ | CH=CH | | |
| 898 | 2-$CH_3$, 4-cyclo-$C_6H_{11}$ | CH=CH | | |
| 899 | 2-$CH_3$, 4-(1,1,3,3-Tetramethylbutyl) | CH=CH | | |
| 900 | 2-$CH_2C_6H_5$ | CH=CH | | |
| 901 | 3-$CH_2C_6H_5$ | CH=CH | | |
| 902 | 4-$CH_2C_6H_5$ | CH=CH | | |
| 903 | 2-$CH_2C_6H_5$, 4-$CH_3$ | CH=CH | | |
| 904 | 2-$CH_3$, 4-$CH_2C_6H_5$ | CH=CH | | |
| 905 | 2-$C_6H_5$ | CH=CH | | |
| 906 | 3-$C_6H_5$ | CH=CH | | |
| 907 | 4-$C_6H_5$ | CH=CH | | |

| Nr. | $R_m$ | X | Fp(°C) | IR($cm^{-1}$) |
|---|---|---|---|---|
| 908 | 4-(2-i-$C_3H_7$-$C_6H_4$) | CH=CH | | |
| 909 | 4-$C_6H_5$, 2,6-$(CH_3)_2$ | CH=CH | | |
| 910 | 2-Cl, 4-$C_6H_5$ | CH=CH | | |
| 911 | 2-Br, 4-$C_6H_5$ | CH=CH | | |
| 912 | 2-$C_6H_5$, 4-Cl | CH=CH | | |
| 913 | 2-$C_6H_5$, 4-Br | CH=CH | | |
| 914 | 2-$CH_2C_6H_5$, 4-Cl | CH=CH | | |
| 915 | 2-$CH_2C_6H_5$, 4-Br | CH=CH | | |
| 916 | 2-Cl, 4-$CH_2C_6H_5$ | CH=CH | | |
| 917 | 2-Br, 4-$CH_2C_6H_5$ | CH=CH | | |
| 918 | 2-cyclo-$C_6H_{11}$, 4-Cl | CH=CH | | |
| 919 | 2-cyclo-$C_6H_{11}$, 4-Br | CH=CH | | |
| 920 | 2-Cl, 4-cyclo-$C_6H_{11}$ | CH=CH | | |
| 921 | 2-Br, 4-cyclo-$C_6H_{11}$ | CH=CH | | |
| 922 | 2-$OCH_3$ | CH=CH | | |
| 923 | 3-$OCH_3$ | CH=CH | | |
| 924 | 4-$OCH_3$ | CH=CH | | |
| 925 | 2,4-$(OCH_3)_2$ | CH=CH | | |
| 926 | 2-$OC_2H_5$ | CH=CH | | |
| 927 | 3-$OC_2H_5$ | CH=CH | | |
| 928 | 4-$OC_2H_5$ | CH=CH | | |
| 929 | 2-$OCH_2C_6H_5$ | CH=CH | | |
| 930 | 3-$OCH_2C_6H_5$ | CH=CH | | |
| 931 | 4-$OCH_2C_6H_5$ | CH=CH | | |
| 932 | 2-O-t-$C_4H_9$ | CH=CH | | |
| 933 | 3-O-t-$C_4H_9$ | CH=CH | | |

| Nr. | $R_m$ | X | Fp(°C) | IR($cm^{-1}$) |
|---|---|---|---|---|
| 934 | 4-O-t-$C_4H_9$ | CH=CH | | |
| 935 | 2-$OC_6H_5$ | CH=CH | | |
| 936 | 3-$OC_6H_5$ | CH=CH | | |
| 937 | 4-$OC_6H_5$ | CH=CH | | |
| 938 | 2-$CF_3$ | CH=CH | | |
| 939 | 3-$CF_3$ | CH=CH | | |
| 940 | 4-$CF_3$ | CH=CH | | |
| 941 | 2-$OCF_3$ | CH=CH | | |
| 942 | 3-$OCF_3$ | CH=CH | | |
| 943 | 4-$OCF_3$ | CH=CH | | |
| 944 | 3-$OCH_2CHF_2$ | CH=CH | | |
| 945 | 3-$OCF_2CHF_2$ | CH=CH | | |
| 946 | 3-$OC_2F_5$ | CH=CH | | |
| 947 | 2-$NO_2$ | CH=CH | | |
| 948 | 3-$NO_2$ | CH=CH | | |
| 949 | 4-$NO_2$ | CH=CH | | |
| 950 | 2-CN | CH=CH | | |
| 951 | 3-CN | CH=CH | | |
| 952 | 4-CN | CH=CH | | |
| 953 | 2-$CH_3$, 3-Cl | CH=CH | | |
| 954 | 2-$CH_3$, 4-Cl | CH=CH | | |
| 955 | 2-$CH_3$, 5-Cl | CH=CH | | |
| 956 | 2-$CH_3$, 6-Cl | CH=CH | | |
| 957 | 2-$CH_3$, 3-F | CH=CH | | |
| 958 | 2-$CH_3$, 4-F | CH=CH | | |
| 959 | 2-$CH_3$, 5-F | CH=CH | | |

| Nr. | $R_m$ | X | Fp(°C) | IR($cm^{-1}$) |
|---|---|---|---|---|
| 960 | 2-$CH_3$, 6-F | CH=CH | | |
| 961 | 2-$CH_3$, 3-Br | CH=CH | | |
| 962 | 2-$CH_3$, 4-Br | CH=CH | | |
| 963 | 2-$CH_3$, 5-Br | CH=CH | | |
| 964 | 2-$CH_3$, 6-Br | CH=CH | | |
| 965 | 2-Cl, 3-$CH_3$ | CH=CH | | |
| 966 | 2-Cl, 4-$CH_3$ | CH=CH | | |
| 967 | 2-Cl, 5-$CH_3$ | CH=CH | | |
| 968 | 2-F, 3-$CH_3$ | CH=CH | | |
| 969 | 2-F, 4-$CH_3$ | CH=CH | | |
| 970 | 2-F, 5-$CH_3$ | CH=CH | | |
| 971 | 2-Br, 3-$CH_3$ | CH=CH | | |
| 972 | 2-Br, 4-$CH_3$ | CH=CH | | |
| 973 | 2-Br, 5-$CH_3$ | CH=CH | | |
| 974 | 3-$CH_3$, 4-Cl | CH=CH | | |
| 975 | 3-$CH_3$, 5-Cl | CH=CH | | |
| 976 | 3-$CH_3$, 4-F | CH=CH | | |
| 977 | 3-$CH_3$, 5-F | CH=CH | | |
| 978 | 3-$CH_3$, 4-Br | CH=CH | | |
| 979 | 3-$CH_3$, 5-Br | CH=CH | | |
| 980 | 3-F, 4-$CH_3$ | CH=CH | | |
| 981 | 3-Cl, 4-$CH_3$ | CH=CH | | |
| 982 | 3-Br, 4-$CH_3$ | CH=CH | | |
| 983 | 2-Cl, 4,5-$(CH_3)_2$ | CH=CH | | |
| 984 | 2-Br, 4,5-$(CH_3)_2$ | CH=CH | | |
| 985 | 2-Cl, 3,5-$(CH_3)_2$ | CH=CH | | |

| Nr. | $R_m$ | X | Fp(°C) | IR($cm^{-1}$) |
|---|---|---|---|---|
| 986 | 2-Br, 3,5-$(CH_3)_2$ | CH=CH | | |
| 987 | 2,6-$Cl_2$, 4-$CH_3$ | CH=CH | | |
| 988 | 2,6-$F_2$, 4-$CH_3$ | CH=CH | | |
| 989 | 2,6-$Br_2$, 4-$CH_3$ | CH=CH | | |
| 990 | 2,4-$Cl_2$, 6-$CH_3$ | CH=CH | | |
| 991 | 2,4-$F_2$, 6-$CH_3$ | CH=CH | | |
| 992 | 2,4-$Br_2$, 6-$CH_3$ | CH=CH | | |
| 993 | 2,6-$(CH_3)_2$, 4-F | CH=CH | | |
| 994 | 2,6-$(CH_3)_2$, 4-Cl | CH=CH | | |
| 995 | 2,6-$(CH_3)_2$, 4-Br | CH=CH | | |
| 996 | 3,5-$(CH_3)_2$, 4-F | CH=CH | | |
| 997 | 3,5-$(CH_3)_2$, 4-Cl | CH=CH | | |
| 998 | 3,5-$(CH_3)_2$, 4-Br | CH=CH | | |
| 999 | 2,3,6-$(CH_3)_3$, 4-F | CH=CH | | |
| 1000 | 2,3,6-$(CH_3)_3$, 4-Cl | CH=CH | | |
| 1001 | 2,3,6-$(CH_3)_3$, 4-Br | CH=CH | | |
| 1002 | 2,4-$(CH_3)_2$, 6-F | CH=CH | | |
| 1003 | 2,4-$(CH_3)_2$, 6-Cl | CH=CH | | |
| 1004 | 2,4-$(CH_3)_2$, 6-Br | CH=CH | | |
| 1005 | 2-i-$C_3H_7$, 4-Cl, 5-$CH_3$ | CH=CH | | |
| 1006 | 2-$CH_3$, 4-Cl, 5-i-$C_3H_7$ | CH=CH | | |
| 1007 | 2-Cl, 3-i-$C_3H_7$ | CH=CH | | |
| 1008 | 2-Cl, 4-i-$C_3H_7$ | CH=CH | | |
| 1009 | 2-Cl, 4-$NO_2$ | CH=CH | | |
| 1010 | 2-$NO_2$, 4-Cl | CH=CH | | |

| Nr. | $R_m$ | X | Fp(°C) | IR($cm^{-1}$) |
|---|---|---|---|---|
| 1011 | 2-$OCH_3$, 5-$NO_2$ | CH=CH | | |
| 1012 | 2,4-$Cl_2$, 5-$NO_2$ | CH=CH | | |
| 1013 | 2,4-$Cl_2$, 6-$NO_2$ | CH=CH | | |
| 1014 | 2,6-$Cl_2$, 4-$NO_2$ | CH=CH | | |
| 1015 | 2,6-$Br_2$, 4-$NO_2$ | CH=CH | | |
| 1016 | 2,6-$J_2$, 4-$NO_2$ | CH=CH | | |
| 1017 | H | $SCH_2$ | | |
| 1018 | 2-F | $SCH_2$ | | |
| 1019 | 3-F | $SCH_2$ | | |
| 1020 | 4-F | $SCH_2$ | | |
| 1021 | 2,3-$F_2$ | $SCH_2$ | | |
| 1022 | 2,4-$F_2$ | $SCH_2$ | | |
| 1023 | 2,4,6-$F_3$ | $SCH_2$ | | |
| 1024 | 2,3,4,5,6-$F_5$ | $SCH_2$ | | |
| 1025 | 2-Cl | $SCH_2$ | | |
| 1026 | 3-Cl | $SCH_2$ | | |
| 1027 | 4-Cl | $SCH_2$ | | |
| 1028 | 2,3-$Cl_2$ | $SCH_2$ | | |
| 1029 | 2,4-$Cl_2$ | $SCH_2$ | | |
| 1030 | 2,5-$Cl_2$ | $SCH_2$ | | |
| 1031 | 2,6-$Cl_2$ | $SCH_2$ | | |

| Nr. | $R_m$ | X | Fp(°C) | IR($cm^{-1}$) |
|---|---|---|---|---|
| 1032 | 3,4-$Cl_2$ | $SCH_2$ | | |
| 1033 | 3,5-$Cl_2$ | $SCH_2$ | | |
| 1034 | 2,3,4-$Cl_3$ | $SCH_2$ | | |
| 1035 | 2,3,5-$Cl_3$ | $SCH_2$ | | |
| 1036 | 2,3,6-$Cl_3$ | $SCH_2$ | | |
| 1037 | 2,4,5-$Cl_3$ | $SCH_2$ | | |
| 1038 | 2,4,6-$Cl_3$ | $SCH_2$ | | |
| 1039 | 3,4,5-$Cl_3$ | $SCH_2$ | | |
| 1040 | 2,3,4,6-$Cl_4$ | $SCH_2$ | | |
| 1041 | 2,3,5,6-$Cl_4$ | $SCH_2$ | | |
| 1042 | 2,3,4,5,6-$Cl_5$ | $SCH_2$ | | |
| 1043 | 2-Br | $SCH_2$ | | |
| 1044 | 3-Br | $SCH_2$ | | |
| 1045 | 4-Br | $SCH_2$ | | |
| 1046 | 2,4-$Br_2$ | $SCH_2$ | | |
| 1047 | 2,5-$Br_2$ | $SCH_2$ | | |
| 1048 | 2,6-$Br_2$ | $SCH_2$ | | |
| 1049 | 2,4,6-$Br_3$ | $SCH_2$ | | |
| 1050 | 2,3,4,5,6-$Br_5$ | $SCH_2$ | | |
| 1051 | 2-J | $SCH_2$ | | |
| 1052 | 3-J | $SCH_2$ | | |
| 1053 | 4-J | $SCH_2$ | | |
| 1054 | 2,4-$J_2$ | $SCH_2$ | | |
| 1055 | 2-Cl, 3-F | $SCH_2$ | | |
| 1056 | 2-Cl, 4-F | $SCH_2$ | | |
| 1057 | 2-Cl, 5-F | $SCH_2$ | | |

| Nr. | $R_m$ | X | Fp(°C) | IR($cm^{-1}$) |
|---|---|---|---|---|
| 1058 | 2-Cl, 6-F | $SCH_2$ | | |
| 1059 | 2-Cl, 3-Br | $SCH_2$ | | |
| 1060 | 2-Cl, 4-Br | $SCH_2$ | | |
| 1061 | 2-Cl, 5-Br | $SCH_2$ | | |
| 1062 | 2-Cl, 6-Br | $SCH_2$ | | |
| 1063 | 2-Br, 3-Cl | $SCH_2$ | | |
| 1064 | 2-Br, 4-Cl | $SCH_2$ | | |
| 1065 | 2-Br, 5-Cl | $SCH_2$ | | |
| 1066 | 2-Br, 3-F | $SCH_2$ | | |
| 1067 | 2-Br, 4-F | $SCH_2$ | | |
| 1068 | 2-Br, 5-F | $SCH_2$ | | |
| 1069 | 2-Br, 6-F | $SCH_2$ | | |
| 1070 | 2-F, 3-Cl | $SCH_2$ | | |
| 1071 | 2-F, 4-Cl | $SCH_2$ | | |
| 1072 | 2-F, 5-Cl | $SCH_2$ | | |
| 1073 | 3-Cl, 4-F | $SCH_2$ | | |
| 1074 | 3-Cl, 5-F | $SCH_2$ | | |
| 1075 | 3-Cl, 4-Br | $SCH_2$ | | |
| 1076 | 3-Cl, 5-Br | $SCH_2$ | | |
| 1077 | 3-F, 4-Cl | $SCH_2$ | | |
| 1078 | 3-F, 4-Br | $SCH_2$ | | |
| 1079 | 3-Br, 4-Cl | $SCH_2$ | | |
| 1080 | 3-Br, 4-F | $SCH_2$ | | |
| 1081 | 2,6-$Cl_2$, 4-Br | $SCH_2$ | | |
| 1082 | 2-$CH_3$ | $SCH_2$ | | |
| 1083 | 3-$CH_3$ | $SCH_2$ | | |

| Nr. | $R_m$ | X | Fp(°C) | IR($cm^{-1}$) |
|---|---|---|---|---|
| 1084 | 4-$CH_3$ | $SCH_2$ | | |
| 1085 | 2,3-$(CH_3)_2$ | $SCH_2$ | | |
| 1086 | 2,4-$(CH_3)_2$ | $SCH_2$ | | |
| 1087 | 2,5-$(CH_3)_2$ | $SCH_2$ | | |
| 1088 | 2,6-$(CH_3)_2$ | $SCH_2$ | | |
| 1089 | 3,4-$(CH_3)_2$ | $SCH_2$ | | |
| 1090 | 3,5-$(CH_3)_2$ | $SCH_2$ | | |
| 1091 | 2,3,4-$(CH_3)_3$ | $SCH_2$ | | |
| 1092 | 2,3,5-$(CH_3)_3$ | $SCH_2$ | | |
| 1093 | 2,3,6-$(CH_3)_3$ | $SCH_2$ | | |
| 1094 | 2,4,5-$(CH_3)_3$ | $SCH_2$ | | |
| 1095 | 2,4,6-$(CH_3)_3$ | $SCH_2$ | | |
| 1096 | 3,4,5-$(CH_3)_3$ | $SCH_2$ | | |
| 1097 | 2,3,4,6-$(CH_3)_4$ | $SCH_2$ | | |
| 1098 | 2,3,5,6-$(CH_3)_4$ | $SCH_2$ | | |
| 1099 | 2,3,4,5,6-$(CH_3)_5$ | $SCH_2$ | | |
| 1100 | 2-$C_2H_5$ | $SCH_2$ | | |
| 1101 | 3-$C_2H_5$ | $SCH_2$ | | |
| 1102 | 4-$C_2H_5$ | $SCH_2$ | | |
| 1103 | 2,4-$(C_2H_5)_2$ | $SCH_2$ | | |
| 1104 | 2,6-$(C_2H_5)_2$ | $SCH_2$ | | |
| 1105 | 3,5-$(C_2H_5)_2$ | $SCH_2$ | | |
| 1106 | 2,4,6-$(C_2H_5)_3$ | $SCH_2$ | | |
| 1107 | 2-n-$C_3H_7$ | $SCH_2$ | | |
| 1108 | 3-n-$C_3H_7$ | $SCH_2$ | | |
| 1109 | 4-n-$C_3H_7$ | $SCH_2$ | | |

| Nr. | $R_m$ | X | Fp(°C) | IR($cm^{-1}$) |
|---|---|---|---|---|
| 1110 | 2-i-$C_2H_7$ | $SCH_2$ | | |
| 1111 | 3-i-$C_2H_7$ | $SCH_2$ | | |
| 1112 | 4-i-$C_3H_7$ | $SCH_2$ | | |
| 1113 | 2,4-(i-$C_3H_7$)$_2$ | $SCH_2$ | | |
| 1114 | 2,6-(i-$C_3H_7$)$_2$ | $SCH_2$ | | |
| 1115 | 3,5-(i-$C_3H_7$)$_2$ | $SCH_2$ | | |
| 1116 | 2,4,6-(i-$C_3H_7$)$_3$ | $SCH_2$ | | |
| 1117 | 2-s-$C_4H_9$ | $SCH_2$ | | |
| 1118 | 3-s-$C_4H_9$ | $SCH_2$ | | |
| 1119 | 4-s-$C_4H_9$ | $SCH_2$ | | |
| 1120 | 2-t-$C_4H_9$ | $SCH_2$ | | |
| 1121 | 3-t-$C_4H_9$ | $SCH_2$ | | |
| 1122 | 4-t-$C_4H_9$ | $SCH_2$ | | |
| 1123 | 2,3-(t-$C_4H_9$)$_2$ | $SCH_2$ | | |
| 1124 | 2,4-(t-$C_4H_9$)$_2$ | $SCH_2$ | | |
| 1125 | 2,5-(t-$C_4H_9$)$_2$ | $SCH_2$ | | |
| 1126 | 2,6-(t-$C_4H_9$)$_2$ | $SCH_2$ | | |
| 1127 | 3,5-(t-$C_4H_9$)$_2$ | $SCH_2$ | | |
| 1128 | 2,4,6-(t-$C_4H_9$)$_3$ | $SCH_2$ | | |
| 1129 | 4-n-$C_9H_{19}$ | $SCH_2$ | | |
| 1130 | 4-n-$C_{12}H_{25}$ | $SCH_2$ | | |
| 1131 | 3-n-$C_{15}H_{31}$ | $SCH_2$ | | |
| 1132 | 4-(1,1,3,3,-Tetramethylbutyl) | $SCH_2$ | | |
| 1133 | 4-(1,1,3,-Trimethylbutyl) | $SCH_2$ | | |
| 1134 | 2-t-$C_4H_9$, 4-$CH_3$ | $SCH_2$ | | |
| 1135 | 2-t-$C_4H_9$, 5-$CH_3$ | $SCH_2$ | | |

| Nr. | $R_m$ | X | Fp(°C) | IR($cm^{-1}$) |
|---|---|---|---|---|
| 1136 | 2,6-(t-$C_4H_9$)$_2$, 4-$CH_3$ | $SCH_2$ | | |
| 1137 | 2-$CH_3$, 4-t-$C_4H_9$ | $SCH_2$ | | |
| 1138 | 2-$CH_3$, 6-t-$C_4H_9$ | $SCH_2$ | | |
| 1139 | 2-$CH_3$, 4-i-$C_3H_7$ | $SCH_2$ | | |
| 1140 | 2-$CH_3$, 5-i-$C_3H_7$ | $SCH_2$ | | |
| 1141 | 3-$CH_3$, 4-i-$C_3H_7$ | $SCH_2$ | | |
| 1142 | 2-i-$C_3H_7$, 5-$CH_3$ | $SCH_2$ | | |
| 1143 | 2,4-(t-$C_4H_9$)$_2$, 6-i-$C_3H_7$ | $SCH_2$ | | |
| 1144 | 2-$C_3H_5$ (=Allyl) | $SCH_2$ | | |
| 1145 | 3-$C_3H_5$ | $SCH_2$ | | |
| 1146 | 4-$C_3H_5$ | $SCH_2$ | | |
| 1147 | 2-$C_3H_5$, 6-$CH_3$ | $SCH_2$ | | |
| 1148 | 2-cyclo-$C_6H_{11}$ | $SCH_2$ | | |
| 1149 | 3-cyclo-$C_6H_{11}$ | $SCH_2$ | | |
| 1150 | 4-cyclo-$C_6H_{11}$ | $SCH_2$ | | |
| 1151 | 2,4-(cyclo-$C_6H_{11}$)$_2$, 6-$CH_3$ | $SCH_2$ | | |
| 1152 | 2-$CH_3$, 4-cyclo-$C_6H_{11}$ | $SCH_2$ | | |
| 1153 | 2-$CH_3$, 4-(1,1,3,3-Tetramethylbutyl) | $SCH_2$ | | |
| 1154 | 2-$CH_2C_6H_5$ | $SCH_2$ | | |
| 1155 | 3-$CH_2C_6H_5$ | $SCH_2$ | | |
| 1156 | 4-$CH_2C_6H_5$ | $SCH_2$ | | |
| 1157 | 2-$CH_2C_6H_5$, 4-$CH_3$ | $SCH_2$ | | |
| 1158 | 2-$CH_3$, 4-$CH_2C_6H_5$ | $SCH_2$ | | |
| 1159 | 2-$C_6H_5$ | $SCH_2$ | | |
| 1160 | 3-$C_6H_5$ | $SCH_2$ | | |
| 1161 | 4-$C_6H_5$ | $SCH_2$ | | |

| Nr. | $R_m$ | X | Fp(°C) | IR($cm^{-1}$) |
|---|---|---|---|---|
| 1162 | 4-(2-i-$C_3H_7$-$C_6H_4$) | $SCH_2$ | | |
| 1163 | 4-$C_6H_5$, 2,6-$(CH_3)_2$ | $SCH_2$ | | |
| 1164 | 2-Cl, 4-$C_6H_5$ | $SCH_2$ | | |
| 1165 | 2-Br, 4-$C_6H_5$ | $SCH_2$ | | |
| 1166 | 2-$C_6H_5$, 4-Cl | $SCH_2$ | | |
| 1167 | 2-$C_6H_5$, 4-Br | $SCH_2$ | | |
| 1168 | 2-$CH_2C_6H_5$, 4-Cl | $SCH_2$ | | |
| 1169 | 2-$CH_2C_6H_5$, 4-Br | $SCH_2$ | | |
| 1170 | 2-Cl, 4-$CH_2C_6H_5$ | $SCH_2$ | | |
| 1171 | 2-Br, 4-$CH_2C_6H_5$ | $SCH_2$ | | |
| 1172 | 2-cyclo-$C_6H_{11}$, 4-Cl | $SCH_2$ | | |
| 1173 | 2-cyclo-$C_6H_{11}$, 4-Br | $SCH_2$ | | |
| 1174 | 2-Cl, 4-cyclo-$C_6H_{11}$ | $SCH_2$ | | |
| 1175 | 2-Br, 4-cyclo-$C_6H_{11}$ | $SCH_2$ | | |
| 1176 | 2-$OCH_3$ | $SCH_2$ | | |
| 1177 | 3-$OCH_3$ | $SCH_2$ | | |
| 1178 | 4-$OCH_3$ | $SCH_2$ | | |
| 1179 | 2,4-$(OCH_3)_2$ | $SCH_2$ | | |
| 1180 | 2-$OC_2H_5$ | $SCH_2$ | | |
| 1181 | 3-$OC_2H_5$ | $SCH_2$ | | |
| 1182 | 4-$OC_2H_5$ | $SCH_2$ | | |
| 1183 | 2-$OCH_2C_6H_5$ | $SCH_2$ | | |
| 1184 | 3-$OCH_2C_6H_5$ | $SCH_2$ | | |
| 1185 | 4-$OCH_2C_6H_5$ | $SCH_2$ | | |
| 1186 | 2-O-t-$C_4H_9$ | $SCH_2$ | | |
| 1187 | 3-O-t-$C_4H_9$ | $SCH_2$ | | |

| Nr. | $R_m$ | X | Fp($^oC$) | IR($cm^{-1}$) |
|---|---|---|---|---|
| 1188 | 4-O-t-$C_4H_9$ | $SCH_2$ | | |
| 1189 | 2-$OC_6H_5$ | $SCH_2$ | | |
| 1190 | 3-$OC_6H_5$ | $SCH_2$ | | |
| 1191 | 4-$OC_6H_5$ | $SCH_2$ | | |
| 1192 | 2-$CF_3$ | $SCH_2$ | | |
| 1193 | 3-$CF_3$ | $SCH_2$ | | |
| 1194 | 4-$CF_3$ | $SCH_2$ | | |
| 1195 | 2-$OCF_3$ | $SCH_2$ | | |
| 1196 | 3-$OCF_3$ | $SCH_2$ | | |
| 1197 | 4-$OCF_3$ | $SCH_2$ | | |
| 1198 | 3-$OCH_2CHF_2$ | $SCH_2$ | | |
| 1199 | 3-$OCF_2CHF_2$ | $SCH_2$ | | |
| 1200 | 3-$OC_2F_5$ | $SCH_2$ | | |
| 1201 | 2-$NO_2$ | $SCH_2$ | | |
| 1202 | 3-$NO_2$ | $SCH_2$ | | |
| 1203 | 4-$NO_2$ | $SCH_2$ | | |
| 1204 | 2-CN | $SCH_2$ | | |
| 1205 | 3-CN | $SCH_2$ | | |
| 1206 | 4-CN | $SCH_2$ | | |
| 1207 | 2-$CH_3$, 3-Cl | $SCH_2$ | | |
| 1208 | 2-$CH_3$, 4-Cl | $SCH_2$ | | |
| 1209 | 2-$CH_3$, 5-Cl | $SCH_2$ | | |
| 1210 | 2-$CH_3$, 6-Cl | $SCH_2$ | | |
| 1211 | 2-$CH_3$, 3-F | $SCH_2$ | | |
| 1212 | 2-$CH_3$, 4-F | $SCH_2$ | | |
| 1213 | 2-$CH_3$, 5-F | $SCH_2$ | | |

| Nr. | $R_m$ | X | Fp(°C) | IR($cm^{-1}$) |
|---|---|---|---|---|
| 1214 | 2-$CH_3$, 6-F | $SCH_2$ | | |
| 1215 | 2-$CH_3$, 3-Br | $SCH_2$ | | |
| 1216 | 2-$CH_3$, 4-Br | $SCH_2$ | | |
| 1217 | 2-$CH_3$, 5-Br | $SCH_2$ | | |
| 1218 | 2-$CH_3$, 6-Br | $SCH_2$ | | |
| 1219 | 2-Cl, 3-$CH_3$ | $SCH_2$ | | |
| 1220 | 2-Cl, 4-$CH_3$ | $SCH_2$ | | |
| 1221 | 2-Cl, 5-$CH_3$ | $SCH_2$ | | |
| 1222 | 2-F, 3-$CH_3$ | $SCH_2$ | | |
| 1223 | 2-F, 4-$CH_3$ | $SCH_2$ | | |
| 1224 | 2-F, 5-$CH_3$ | $SCH_2$ | | |
| 1225 | 2-Br, 3-$CH_3$ | $SCH_2$ | | |
| 1226 | 2-Br, 4-$CH_3$ | $SCH_2$ | | |
| 1227 | 2-Br, 5-$CH_3$ | $SCH_2$ | | |
| 1228 | 3-$CH_3$, 4-Cl | $SCH_2$ | | |
| 1229 | 3-$CH_3$, 5-Cl | $SCH_2$ | | |
| 1230 | 3-$CH_3$, 4-F | $SCH_2$ | | |
| 1231 | 3-$CH_3$, 5-F | $SCH_2$ | | |
| 1232 | 3-$CH_3$, 4-Br | $SCH_2$ | | |
| 1233 | 3-$CH_3$, 5-Br | $SCH_2$ | | |
| 1234 | 3-F, 4-$CH_3$ | $SCH_2$ | | |
| 1235 | 3-Cl, 4-$CH_3$ | $SCH_2$ | | |
| 1236 | 3-Br, 4-$CH_3$ | $SCH_2$ | | |
| 1237 | 2-Cl, 4,5-$(CH_3)_2$ | $SCH_2$ | | |
| 1238 | 2-Br, 4,5-$(CH_3)_2$ | $SCH_2$ | | |
| 1239 | 2-Cl, 3,5-$(CH_3)_2$ | $SCH_2$ | | |

| Nr. | $R_m$ | X | Fp(°C) | IR($cm^{-1}$) |
|---|---|---|---|---|
| 1240 | 2-Br, 3,5-$(CH_3)_2$ | $SCH_2$ | | |
| 1241 | 2,6-$Cl_2$, 4-$CH_3$ | $SCH_2$ | | |
| 1242 | 2,6-$F_2$, 4-$CH_3$ | $SCH_2$ | | |
| 1243 | 2,6-$Br_2$, 4-$CH_3$ | $SCH_2$ | | |
| 1244 | 2,4-$Cl_2$, 6-$CH_3$ | $SCH_2$ | | |
| 1245 | 2,4-$F_2$, 6-$CH_3$ | $SCH_2$ | | |
| 1246 | 2,4-$Br_2$, 6-$CH_3$ | $SCH_2$ | | |
| 1247 | 2,6-$(CH_3)_2$, 4-F | $SCH_2$ | | |
| 1248 | 2,6-$(CH_3)_2$, 4-Cl | $SCH_2$ | | |
| 1249 | 2,6-$(CH_3)_2$, 4-Br | $SCH_2$ | | |
| 1250 | 3,5-$(CH_3)_2$, 4-F | $SCH_2$ | | |
| 1251 | 3,5-$(CH_3)_2$, 4-Cl | $SCH_2$ | | |
| 1252 | 3,5-$(CH_3)_2$, 4-Br | $SCH_2$ | | |
| 1253 | 2,3,6-$(CH_3)_3$, 4-F | $SCH_2$ | | |
| 1254 | 2,3,6-$(CH_3)_3$, 4-Cl | $SCH_2$ | | |
| 1255 | 2,3,6-$(CH_3)_3$, 4-Br | $SCH_2$ | | |
| 1256 | 2,4-$(CH_3)_2$, 6-F | $SCH_2$ | | |
| 1257 | 2,4-$(CH_3)_2$, 6-Cl | $SCH_2$ | | |
| 1258 | 2,4-$(CH_3)_2$, 6-Br | $SCH_2$ | | |
| 1259 | 2-i-$C_3H_7$, 4-Cl, 5-$CH_3$ | $SCH_2$ | | |
| 1260 | 2-$CH_3$, 4-Cl, 5-i-$C_3H_7$ | $SCH_2$ | | |
| 1261 | 2-Cl, 3-i-$C_3H_7$ | $SCH_2$ | | |
| 1262 | 2-Cl, 4-i-$C_3H_7$ | $SCH_2$ | | |
| 1263 | 2-Cl, 4-$NO_2$ | $SCH_2$ | | |
| 1264 | 2-$NO_2$, 4-Cl | $SCH_2$ | | |

| Nr. | $R_m$ | X | Fp(°C) | IR($cm^{-1}$) |
|---|---|---|---|---|
| 1265 | 2-$OCH_3$, 5-$NO_2$ | $SCH_2$ | | |
| 1266 | 2,4-$Cl_2$, 5-$NO_2$ | $SCH_2$ | | |
| 1267 | 2,4-$Cl_2$, 6-$NO_2$ | $SCH_2$ | | |
| 1268 | 2,6-$Cl_2$, 4-$NO_2$ | $SCH_2$ | | |
| 1269 | 2,6-$Br_2$, 4-$NO_2$ | $SCH_2$ | | |
| 1270 | 2,6-$J_2$, 4-$NO_2$ | $SCH_2$ | | |
| 1271 | H | O | | |
| 1272 | 2-F | O | | |
| 1273 | 3-F | O | | |
| 1274 | 4-F | O | | |
| 1275 | 2,3-$F_2$ | O | | |
| 1276 | 2,4-$F_2$ | O | | |
| 1277 | 2,4,6-$F_3$ | O | | |
| 1278 | 2,3,4,5,6-$F_5$ | O | | |
| 1279 | 2-Cl | O | | |
| 1280 | 3-Cl | O | | |
| 1281 | 4-Cl | O | | |
| 1282 | 2,3-$Cl_2$ | O | | |
| 1283 | 2,4-$Cl_2$ | O | | |
| 1284 | 2,5-$Cl_2$ | O | | |
| 1285 | 2,6-$Cl_2$ | O | | |

| Nr. | $R_m$ | X | Fp(°C) | IR($cm^{-1}$) |
|---|---|---|---|---|
| 1286 | 3,4-$Cl_2$ | O | | |
| 1287 | 3,5-$Cl_2$ | O | | |
| 1288 | 2,3,4-$Cl_3$ | O | | |
| 1289 | 2,3,5-$Cl_3$ | O | | |
| 1290 | 2,3,6-$Cl_3$ | O | | |
| 1291 | 2,4,5-$Cl_3$ | O | | |
| 1292 | 2,4,6-$Cl_3$ | O | | |
| 1293 | 3,4,5-$Cl_3$ | O | | |
| 1294 | 2,3,4,6-$Cl_4$ | O | | |
| 1295 | 2,3,5,6-$Cl_4$ | O | | |
| 1296 | 2,3,4,5,6-$Cl_5$ | O | | |
| 1297 | 2-Br | O | | |
| 1298 | 3-Br | O | | |
| 1299 | 4-Br | O | | |
| 1300 | 2,4-$Br_2$ | O | | |
| 1301 | 2,5-$Br_2$ | O | | |
| 1302 | 2,6-$Br_2$ | O | | |
| 1303 | 2,4,6-$Br_3$ | O | | |
| 1304 | 2,3,4,5,6-$Br_5$ | O | | |
| 1305 | 2-J | O | | |
| 1306 | 3-J | O | | |
| 1307 | 4-J | O | | |
| 1308 | 2,4-$J_2$ | O | | |
| 1309 | 2-Cl, 3-F | O | | |
| 1310 | 2-Cl, 4-F | O | | |
| 1311 | 2-Cl, 5-F | O | | |

| Nr. | $R_m$ | X | Fp(°C) | IR($cm^{-1}$) |
|---|---|---|---|---|
| 1312 | 2-Cl, 6-F | O | | |
| 1313 | 2-Cl, 3-Br | O | | |
| 1314 | 2-Cl, 4-Br | O | | |
| 1315 | 2-Cl, 5-Br | O | | |
| 1316 | 2-Cl, 6-Br | O | | |
| 1317 | 2-Br, 3-Cl | O | | |
| 1318 | 2-Br, 4-Cl | O | | |
| 1319 | 2-Br, 5-Cl | O | | |
| 1320 | 2-Br, 3-F | O | | |
| 1321 | 2-Br, 4-F | O | | |
| 1322 | 2-Br, 5-F | O | | |
| 1323 | 2-Br, 6-F | O | | |
| 1324 | 2-F, 3-Cl | O | | |
| 1325 | 2-F, 4-Cl | O | | |
| 1326 | 2-F, 5-Cl | O | | |
| 1327 | 3-Cl, 4-F | O | | |
| 1328 | 3-Cl, 5-F | O | | |
| 1329 | 3-Cl, 4-Br | O | | |
| 1330 | 3-Cl, 5-Br | O | | |
| 1331 | 3-F, 4-Cl | O | | |
| 1332 | 3-F, 4-Br | O | | |
| 1333 | 3-Br, 4-Cl | O | | |
| 1334 | 3-Br, 4-F | O | | |
| 1335 | 2,6-$Cl_2$, 4-Br | O | | |
| 1336 | 2-$CH_3$ | O | | |
| 1337 | 3-$CH_3$ | O | | |

| Nr. | $R_m$ | X | Fp(°C) | IR($cm^{-1}$) |
|---|---|---|---|---|
| 1338 | 4-$CH_3$ | O | | |
| 1339 | 2,3-$(CH_3)_2$ | O | | |
| 1340 | 2,4-$(CH_3)_2$ | O | | |
| 1341 | 2,5-$(CH_3)_2$ | O | | |
| 1342 | 2,6-$(CH_3)_2$ | O | | |
| 1343 | 3,4-$(CH_3)_2$ | O | | |
| 1344 | 3,5-$(CH_3)_2$ | O | | |
| 1345 | 2,3,4-$(CH_3)_3$ | O | | |
| 1346 | 2,3,5-$(CH_3)_3$ | O | | |
| 1347 | 2,3,6-$(CH_3)_3$ | O | | |
| 1348 | 2,4,5-$(CH_3)_3$ | O | | |
| 1349 | 2,4,6-$(CH_3)_3$ | O | | |
| 1350 | 3,4,5-$(CH_3)_3$ | O | | |
| 1351 | 2,3,4,6-$(CH_3)_4$ | O | | |
| 1352 | 2,3,5,6-$(CH_3)_4$ | O | | |
| 1353 | 2,3,4,5,6-$(CH_3)_5$ | O | | |
| 1354 | 2-$C_2H_5$ | O | | |
| 1355 | 3-$C_2H_5$ | O | | |
| 1356 | 4-$C_2H_5$ | O | | |
| 1357 | 2,4-$(C_2H_5)_2$ | O | | |
| 1358 | 2,6-$(C_2H_5)_2$ | O | | |
| 1359 | 3,5-$(C_2H_5)_2$ | O | | |
| 1360 | 2,4,6-$(C_2H_5)_3$ | O | | |
| 1361 | 2-n-$C_3H_7$ | O | | |
| 1362 | 3-n-$C_3H_7$ | O | | |
| 1363 | 4-n-$C_3H_7$ | O | | |

| Nr. | $R_m$ | X | Fp(°C) | IR($cm^{-1}$) |
|---|---|---|---|---|
| 1364 | 2-i-$C_2H_7$ | O | | |
| 1365 | 3-i-$C_2H_7$ | O | | |
| 1366 | 4-i-$C_3H_7$ | O | | |
| 1367 | 2,4-(i-$C_3H_7$)$_2$ | O | | |
| 1368 | 2,6-(i-$C_3H_7$)$_2$ | O | | |
| 1369 | 3,5-(i-$C_3H_7$)$_2$ | O | | |
| 1370 | 2,4,6-(i-$C_3H_7$)$_3$ | O | | |
| 1371 | 2-s-$C_4H_9$ | O | | |
| 1372 | 3-s-$C_4H_9$ | O | | |
| 1373 | 4-s-$C_4H_9$ | O | | |
| 1374 | 2-t-$C_4H_9$ | O | | |
| 1375 | 3-t-$C_4H_9$ | O | | |
| 1376 | 4-t-$C_4H_9$ | O | | |
| 1377 | 2,3-(t-$C_4H_9$)$_2$ | O | | |
| 1378 | 2,4-(t-$C_4H_9$)$_2$ | O | | |
| 1379 | 2,5-(t-$C_4H_9$)$_2$ | O | | |
| 1380 | 2,6-(t-$C_4H_9$)$_2$ | O | | |
| 1381 | 3,5-(t-$C_4H_9$)$_2$ | O | | |
| 1382 | 2,4,6-(t-$C_4H_9$)$_3$ | O | | |
| 1383 | 4-n-$C_9H_{19}$ | O | | |
| 1384 | 4-n-$C_{12}H_{25}$ | O | | |
| 1385 | 3-n-$C_{15}H_{31}$ | O | | |
| 1386 | 4-(1,1,3,3,-Tetramethylbutyl) | O | | |
| 1387 | 4-(1,1,3,-Trimethylbutyl) | O | | |
| 1388 | 2-t-$C_4H_9$, 4-$CH_3$ | O | | |
| 1389 | 2-t-$C_4H_9$, 5-$CH_3$ | O | | |

| Nr. | $R_m$ | X | Fp($^oC$) | IR($cm^{-1}$) |
|---|---|---|---|---|
| 1390 | 2,6-(t-$C_4H_9$)$_2$, 4-$CH_3$ | O | | |
| 1391 | 2-$CH_3$, 4-t-$C_4H_9$ | O | | |
| 1392 | 2-$CH_3$, 6-t-$C_4H_9$ | O | | |
| 1393 | 2-$CH_3$, 4-i-$C_3H_7$ | O | | |
| 1394 | 2-$CH_3$, 5-i-$C_3H_7$ | O | | |
| 1395 | 3-$CH_3$, 4-i-$C_3H_7$ | O | | |
| 1396 | 2-i-$C_3H_7$, 5-$CH_3$ | O | | |
| 1397 | 2,4-(t-$C_4H_9$)$_2$, 6-i-$C_3H_7$ | O | | |
| 1398 | 2-$C_3H_5$ (=Allyl) | O | | |
| 1399 | 3-$C_3H_5$ | O | | |
| 1400 | 4-$C_3H_5$ | O | | |
| 1401 | 2-$C_3H_5$, 6-$CH_3$ | O | | |
| 1402 | 2-cyclo-$C_6H_{11}$ | O | | |
| 1403 | 3-cyclo-$C_6H_{11}$ | O | | |
| 1404 | 4-cyclo-$C_6H_{11}$ | O | | |
| 1405 | 2,4-(cyclo-$C_6H_{11}$)$_2$, 6-$CH_3$ | O | | |
| 1406 | 2-$CH_3$, 4-cyclo-$C_6H_{11}$ | O | | |
| 1407 | 2-$CH_3$, 4-(1,1,3,3-Tetramethylbutyl) | O | | |
| 1408 | 2-$CH_2C_6H_5$ | O | | |
| 1409 | 3-$CH_2C_6H_5$ | O | | |
| 1410 | 4-$CH_2C_6H_5$ | O | | |
| 1411 | 2-$CH_2C_6H_5$, 4-$CH_3$ | O | | |
| 1412 | 2-$CH_3$, 4-$CH_2C_6H_5$ | O | | |
| 1413 | 2-$C_6H_5$ | O | | |
| 1414 | 3-$C_6H_5$ | O | | |
| 1415 | 4-$C_6H_5$ | O | | |

| Nr. | $R_m$ | X | Fp(°C) | IR($cm^{-1}$) |
|---|---|---|---|---|
| 1416 | 4-(2-i-$C_3H_7$-$C_6H_4$) | O | | |
| 1417 | 4-$C_6H_5$, 2,6-$(CH_3)_2$ | O | | |
| 1418 | 2-Cl, 4-$C_6H_5$ | O | | |
| 1419 | 2-Br, 4-$C_6H_5$ | O | | |
| 1420 | 2-$C_6H_5$, 4-Cl | O | | |
| 1421 | 2-$C_6H_5$, 4-Br | O | | |
| 1422 | 2-$CH_2C_6H_5$, 4-Cl | O | | |
| 1423 | 2-$CH_2C_6H_5$, 4-Br | O | | |
| 1424 | 2-Cl, 4-$CH_2C_6H_5$ | O | | |
| 1425 | 2-Br, 4-$CH_2C_6H_5$ | O | | |
| 1426 | 2-cyclo-$C_6H_{11}$, 4-Cl | O | | |
| 1427 | 2-cyclo-$C_6H_{11}$, 4-Br | O | | |
| 1428 | 2-Cl, 4-cyclo-$C_6H_{11}$ | O | | |
| 1429 | 2-Br, 4-cyclo-$C_6H_{11}$ | O | | |
| 1430 | 2-$OCH_3$ | O | | |
| 1431 | 3-$OCH_3$ | O | | |
| 1432 | 4-$OCH_3$ | O | | |
| 1433 | 2,4-$(OCH_3)_2$ | O | | |
| 1434 | 2-$OC_2H_5$ | O | | |
| 1435 | 3-$OC_2H_5$ | O | | |
| 1436 | 4-$OC_2H_5$ | O | | |
| 1437 | 2-$OCH_2C_6H_5$ | O | | |
| 1438 | 3-$OCH_2C_6H_5$ | O | | |
| 1439 | 4-$OCH_2C_6H_5$ | O | | |
| 1440 | 2-O-t-$C_4H_9$ | O | | |
| 1441 | 3-O-t-$C_4H_9$ | O | | |

| Nr. | $R_m$ | X | Fp(°C) | IR($cm^{-1}$) |
|---|---|---|---|---|
| 1442 | 4-O-t-$C_4H_9$ | O | | |
| 1443 | 2-$OC_6H_5$ | O | | |
| 1444 | 3-$OC_6H_5$ | O | | |
| 1445 | 4-$OC_6H_5$ | O | | |
| 1446 | 2-$CF_3$ | O | | |
| 1447 | 3-$CF_3$ | O | | |
| 1448 | 4-$CF_3$ | O | | |
| 1449 | 2-$OCF_3$ | O | | |
| 1450 | 3-$OCF_3$ | O | | |
| 1451 | 4-$OCF_3$ | O | | |
| 1452 | 3-$OCH_2CHF_2$ | O | | |
| 1453 | 3-$OCF_2CHF_2$ | O | | |
| 1454 | 3-$OC_2F_5$ | O | | |
| 1455 | 2-$NO_2$ | O | | |
| 1456 | 3-$NO_2$ | O | | |
| 1457 | 4-$NO_2$ | O | | |
| 1458 | 2-CN | O | | |
| 1459 | 3-CN | O | | |
| 1460 | 4-CN | O | | |
| 1461 | 2-$CH_3$, 3-Cl | O | | |
| 1462 | 2-$CH_3$, 4-Cl | O | | |
| 1463 | 2-$CH_3$, 5-Cl | O | | |
| 1464 | 2-$CH_3$, 6-Cl | O | | |
| 1465 | 2-$CH_3$, 3-F | O | | |
| 1466 | 2-$CH_3$, 4-F | O | | |
| 1467 | 2-$CH_3$, 5-F | O | | |

| Nr. | $R_m$ | X | Fp($^oC$) | IR($cm^{-1}$) |
|---|---|---|---|---|
| 1468 | 2-$CH_3$, 6-F | O | | |
| 1469 | 2-$CH_3$, 3-Br | O | | |
| 1470 | 2-$CH_3$, 4-Br | O | | |
| 1471 | 2-$CH_3$, 5-Br | O | | |
| 1472 | 2-$CH_3$, 6-Br | O | | |
| 1473 | 2-Cl, 3-$CH_3$ | O | | |
| 1474 | 2-Cl, 4-$CH_3$ | O | | |
| 1475 | 2-Cl, 5-$CH_3$ | O | | |
| 1476 | 2-F, 3-$CH_3$ | O | | |
| 1477 | 2-F, 4-$CH_3$ | O | | |
| 1478 | 2-F, 5-$CH_3$ | O | | |
| 1479 | 2-Br, 3-$CH_3$ | O | | |
| 1480 | 2-Br, 4-$CH_3$ | O | | |
| 1481 | 2-Br, 5-$CH_3$ | O | | |
| 1482 | 3-$CH_3$, 4-Cl | O | | |
| 1483 | 3-$CH_3$, 5-Cl | O | | |
| 1484 | 3-$CH_3$, 4-F | O | | |
| 1485 | 3-$CH_3$, 5-F | O | | |
| 1486 | 3-$CH_3$, 4-Br | O | | |
| 1487 | 3-$CH_3$, 5-Br | O | | |
| 1488 | 3-F, 4-$CH_3$ | O | | |
| 1489 | 3-Cl, 4-$CH_3$ | O | | |
| 1490 | 3-Br, 4-$CH_3$ | O | | |
| 1491 | 2-Cl, 4,5-$(CH_3)_2$ | O | | |
| 1492 | 2-Br, 4,5-$(CH_3)_2$ | O | | |
| 1493 | 2-Cl, 3,5-$(CH_3)_2$ | O | | |

| Nr. | $R_m$ | X | Fp(°C) | IR($cm^{-1}$) |
|---|---|---|---|---|
| 1494 | 2-Br, 3,5-$(CH_3)_2$ | O | | |
| 1495 | 2,6-$Cl_2$, 4-$CH_3$ | O | | |
| 1496 | 2,6-$F_2$, 4-$CH_3$ | O | | |
| 1497 | 2,6-$Br_2$, 4-$CH_3$ | O | | |
| 1498 | 2,4-$Cl_2$, 6-$CH_3$ | O | | |
| 1499 | 2,4-$F_2$, 6-$CH_3$ | O | | |
| 1500 | 2,4-$Br_2$, 6-$CH_3$ | O | | |
| 1501 | 2,6-$(CH_3)_2$, 4-F | O | | |
| 1502 | 2,6-$(CH_3)_2$, 4-Cl | O | | |
| 1503 | 2,6-$(CH_3)_2$, 4-Br | O | | |
| 1504 | 3,5-$(CH_3)_2$, 4-F | O | | |
| 1505 | 3,5-$(CH_3)_2$, 4-Cl | O | | |
| 1506 | 3,5-$(CH_3)_2$, 4-Br | O | | |
| 1507 | 2,3,6-$(CH_3)_3$, 4-F | O | | |
| 1508 | 2,3,6-$(CH_3)_3$, 4-Cl | O | | |
| 1509 | 2,3,6-$(CH_3)_3$, 4-Br | O | | |
| 1510 | 2,4-$(CH_3)_2$, 6-F | O | | |
| 1511 | 2,4-$(CH_3)_2$, 6-Cl | O | | |
| 1512 | 2,4-$(CH_3)_2$, 6-Br | O | | |
| 1513 | 2-i-$C_3H_7$, 4-Cl, 5-$CH_3$ | O | | |
| 1514 | 2-$CH_3$, 4-Cl, 5-i-$C_3H_7$ | O | | |
| 1515 | 2-Cl, 3-i-$C_3H_7$ | O | | |
| 1516 | 2-Cl, 4-i-$C_3H_7$ | O | | |
| 1517 | 2-Cl, 4-$NO_2$ | O | | |
| 1518 | 2-$NO_2$, 4-Cl | O | | |

| Nr. | $R_m$ | X | Fp(°C) | IR($cm^{-1}$) |
|---|---|---|---|---|
| 1519 | 2-$OCH_3$, 5-$NO_2$ | O | | |
| 1520 | 2,4-$Cl_2$, 5-$NO_2$ | O | | |
| 1521 | 2,4-$Cl_2$, 6-$NO_2$ | O | | |
| 1522 | 2,6-$Cl_2$, 4-$NO_2$ | O | | |
| 1523 | 2,6-$Br_2$, 4-$NO_2$ | O | | |
| 1524 | 2,6-$J_2$, 4-$NO_2$ | O | | |
| 1525 | *1) | $OCH_2$ | | |
| 1526 | *2) | $OCH_2$ | | |
| 1527 | *1) | $CH_2O$ | | |
| 1528 | *2) | $CH_2O$ | | |

*1) 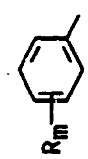 = 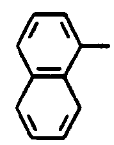

*2) 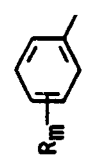 = 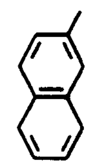

Die neuen Verbindungen zeichnen sich, allgemein ausgedrückt, durch eine hervorragende Wirksamkeit gegen ein breites Spektrum von pflanzenpathogenen Pilzen, insbesondere aus der Klasse der Ascomyceten und Basidiomyceten, aus. Sie sind zum Teil systemisch wirksam und können als Blatt- und Bodenfungizide eingesetzt werden.

Besonders interessant sind die fungiziden Verbindungen für die Bekämpfung einer Vielzahl von Pilzen an verschiedenen Kulturpflanzen oder ihren Samen, insbesondere Weizen, Roggen, Gerste, Hafer, Reis, Mais, Rasen, Baumwolle, Soja, Kaffee, Zuckerrohr, Obst und Zierpflanzen im Gartenbau, Weinbau sowie Gemüse - wie Gurken, Bohnen und Kürbisgewächse -.

Die neuen Verbindungen sind insbesondere geeignet zur Bekämpfung folgender Pflanzenkrankheiten:

Erysiphe graminis (echter Mehltau) in Getreide,
Erysiphe cichoracearum und Sphaerotheca fuliginea an Kürbisgewächsen,
Podosphaera leucotricha an Äpfeln,
Uncinula necator an Reben,
Puccinia-Arten an Getreide,
Rhizoctonia-Arten an Baumwolle und Rasen,
Ustilago-Arten an Getreide und Zuckerrohr,
Venturia inaequalis (Schorf) an Äpfeln,
Helminthosporium-Arten an Getreide,

Septoria nodorum an Weizen,
Botrytis cinerea (Grauschimmel) an Erdbeeren, Reben,
Cercospora arachidicola an Erdnüssen,
Pseudocercosporella herpotrichoides an Weizen, Gerste,
Pyricularia oryzae an Reis,
Phytophthora infestans an Kartoffeln und Tomaten,
Fusarium- und Verticillium-Arten an verschiedenen Pflanzen,
Plasmopara viticola an Reben,
Alternaria-Arten an Gemüse und Obst.

Die Verbindungen werden angewendet, indem man die Pflanzen mit den Wirkstoffen besprüht oder bestäubt oder die Samen der Pflanzen mit den Wirkstoffen behandelt. Die Anwendung erfolgt vor oder nach der Infektion der Pflanzen oder Samen durch die Pilze. Es werden die Pilze oder die vor pilzbefall zu schützenden Pflanzen, Saatgüter, Materialien oder der Erdboden mit einer fungizid wirksamen Menge des Wirkstoffs behandelt.

Die neuen Substanzen können in die üblichen Formulierungen übergeführt werden, wie Lösungen, Emulsionen, Suspensionen, Stäube, Pulver, Pasten und Granulate. Die Anwendungsformen richten sich ganz nach den Verwendungszwecken; sie sollen in jedem Fall eine feine und gleichmäßige Verteilung der wirksamen Substanz gewährleisten. Die Formulierungen werden in bekannter Weise hergestellt, z.B. durch Verstrecken des Wirkstoffs mit Lösungsmitteln und/oder Trägerstoffen, gegebenenfalls unter Verwendung von Emulgiermitteln und Dispergiermitteln, wobei im Falle der Benutzung von Wasser als Verdünnungsmittel auch andere organische Lösungsmittel als Hilfslösungsmittel verwendet werden können. Als Hilfsstoffe kommen dafür im wesentlichen in Frage: Lösungsmittel wie Aromaten (z.B. Xylol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B. Ethanolamin, Dimethylformamid) und Wasser; Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel, wie nichtionogene und anionische Emulgatoren (z.B. Polyoxyethylen-Fettalkohol-Ether, Alkylsulfonate und Arylsulfonate) und Dispergiermittel, wie Lignin-Sulfitablaugen und Methylcellulose.

Die fungiziden Mittel enthalten im allgemeinen zwischen 0,1 und 95, vorzugsweise zwischen 0,5 und 90 Gew.% Wirkstoff.

Die Aufwandmengen liegen je nach Art des gewünschten Effektes zwischen 0,02 und 3 kg Wirkstoff oder mehr je ha. Die neuen Verbindungen können auch im Materialschutz (Holzschutz) eingesetzt werden, z.B. gegen Paecilomyces variotii. Bei der Saatgutbehandlung werden im allgemeinen Wirkstoffmengen von 0,001 bis 50 g, vorzugsweise 0,01 bis 10 g, je kg Saatgut benötigt.

Die Mittel bzw. die daraus hergestellten gebrauchsfertigen Zubereitungen, wie Lösungen, Emulsionen, Suspensionen, Pulver, Stäube, Pasten oder Granulate werden in bekannter Weise angewendet, beispielsweiise durch Versprühen, Vernebeln, Verstäuben, Verstreuen, Beizen oder Gießen.

Beispiele für solche Zubereitungen sind:

I. Man vermischt 90 Gew.-Teile der Verbindung Nr. 4 mit 10 Gew.-Teilen N-Methyl-$\alpha$-pyrrolidon und erhält eine Lösung, die zur Anwendung in Form kleinster Tropfen geeignet ist.

II. 20 Gew.-Teile der Verbindung Nr. 11 werden in einer Mischung gelöst, die aus 80 Gew.-Teilen Xylol, 10 Gew.-Teilen des Anlagerungsproduktes von 8 bis 10 Mol Ethylenoxid an 1 Mol Ölsäure-N-monoethanolamid, 5 Gew.-Teilen Calciumsalz der Dodecylbenzolsulfonsäure und 5 Gew.-Teilen des Anlagerungsproduktes und 40 Mol Ethylenoxid an 1 Mol Ricinusöl besteht. Durch Ausgießen und feines Verteilen der Lösung in Wasser erhält man eine wäßrige Dispersion.

III. 20 Gew.-Teile der Verbindung Nr. 66 werden in einer Mischung gelöst, die aus 40 Gew.-Teilen Cyclohexanon, 30 Gew.-Teilen Isobutanol, 20 Gew.-Teilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

IV. 20 Gew.-Teile der Verbindung Nr. 4 werden in einer Mischung gelöst, die aus 25 Gew.-Teilen Cyclohexanol, 65 Gew.-Teilen einer Mineralölfraktion vom Siedepunkt 210 bis 280°C und 10 Gew.-Teilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

V. 80 Gew.-Teile der Verbindung Nr. 11 werden mit 3 Gew.-Teilen des Natriumsalzes der Diisobutylnaphthalin-$\alpha$-sulfonsäure, 10 Gew.-Teilen des Natriumsalzes einer Ligninsulfonsäure aus einer Sulfitablauge und 7 Gew.-Teilen pulverförmigem Kieselsäuregel gut vermischt und in einer Hammermühle vermahlen. Durch feines Verteilen der Mischung in Wasser erhält man eine Spritzbrühe.

VI. 3 Gew.-Teile der Verbindung Nr. 66 werden mit 97 Gew.-Teilen feinteiligem Kaolin innig vermischt. Man erhält auf diese Weise ein Stäubemittel, das 3 Gew.% des Wirkstoffs enthält.

VII. 30 Gew.-Teile der Verbindung Nr. 4 werden mit einer Mischung aus 92 Gew.-Teilen pulverförmigem Kieselsäuregel und 8 Gew.-Teilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 40 Gew.-Teile der Verbindung Nr. 11 werden mit 10 Gew.-Teilen Natriumsalz eines Phenolsulfonsäureharnstoff-formaldehyd-Kondensates, 2 Gew.-Teilen Kieselgel und 48 Gew.-Tellen Wasser innig vermischt. Man erhält eine stabile wäßrige Dispersion. Durch Verdünnen mit Wasser erhält man eine wäßrige Dispersion.

IX. 20 Gew.-Teile der Verbindung Nr. 66 werden mit 2 Gew.-Teilen Calciumsalz der Dodecylbenzolsulfonsäure, 8 Gew.-Teilen Fettalkoholpolyglykolether, 2 Gew.-Teilen Natriumsalz eines Phenolsulfonsäure-harnstoffformaldehyd-Kondensats und 68 Gew.-Teilen eines paraffinischen Mineralöls innig vermischt. Man erhält eine stabile ölige Dispersion.

Die erfindungsgemäßen Mittel können in diesen Anwendungsformen auch zusammen mit anderen Wirkstoffen vorliegen, wie z.B. Herbiziden, Insektiziden, Wachstumsregulatoren und Fungiziden, oder auch mit Düngemitteln vermischt und ausgebracht werden. Beim Vermischen mit Fungiziden erhält man dabei in vielen Fällen eine Vergrößerung des fungiziden Wirkungsspektrums.

Anwendungsbeispiel

Als Vergleichswirkstoff wurde 2-(2-Methylbenzyloxy)-phenylglyoxyl säuremethylester-O-methyloxim (A) - bekannt aus EP 253 213 - benutzt.

Wirksamkeit gegen Pyrenophora teres

Gerstenkeimlinge der Sorte "Igri" wurden im Zweiblattstadium mit wäßrigen Suspensionen, die 80 % Wirkstoff und 20 % Emulgator in der Trockensubstanz enthielten, tropfnaß gespritzt. Nach 24 Stunden wurden die Pflanzen mit einer Sporensuspension des Pilzes Pyrenophora teres inokuliert und für 48 Stunden in eine Klimakammer mit hoher Luftfeuchtigkeit bei 18°C gestellt. Anschließend wurden die Pflanzen im Gewächshaus bei 20 - 22°C und 70 % relativer Luftfeuchtigkeit für weitere 5 Tage kultiviert. Dann wurde das Ausmaß des Befalls der Blätter ermittelt.

Das Ergebnis zeigt, daß die Wirkstoffe 4, 11 und 66 bei der Anwendung als 0,05 %ige (Gew.%) Spritzbrühe eine bessere fungizide Wirkung zeigen (90 %) als der bekannte Vergleichswirkstoff A (60 %).

## Patentansprüche

**1.** 3-Methoximinopropionsäureester der Formel I

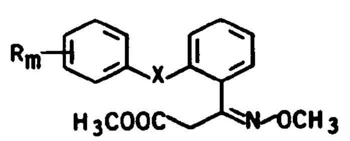

(I)

in der

R (m = 1 bis 5) gleiche oder verschiedene Substituenten Wasserstoff, Halogen, Cyano, Nitro, $C_1$-$C_{15}$-Alkyl, $C_3$-$C_6$-Cycloalkyl, $C_3$-$C_6$-Alkenyl, $C_1$-$C_4$-Alkoxy, $C_1$-$C_2$-Halogenalkyl, $C_1$-$C_2$-Halogenalkoxy, gegebenenfalls substituiertes Phenyl, gegebenenfalls substituiertes Phenoxy, gegebenenfalls substituiertes Benzyl oder gegebenenfalls substituiertes Benzyloxy bedeutet, oder in der die Gruppe

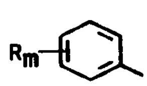

a-Naphthyl oder β-Naphthyl bedeutet und

X

Methylenoxy, Oxymethylen, Ethylen, Ethenylen, Thiomethylen oder Sauerstoff bedeutet.

2. Verfahren zur Bekämpfung von Pilzen, dadurch gekennzeichnet, daß man die Pilze oder die vom Pilzbefall bedrohten Materialien, Pflanzen, Saatgüter oder den Erdboden mit einer fungizid wirksamen Menge einer Verbindung der Formel I behandelt

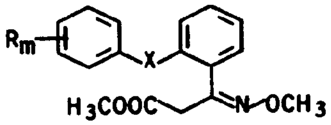

(I)

in der

R (m = 1 bis 5) gleiche oder verschiedene Substituenten Wasserstoff, Halogen, Cyano, Nitro, $C_1$-$C_{15}$-Alkyl, $C_3$-$C_6$-Cycloalkyl, $C_3$-$C_6$-Alkenyl, $C_1$-$C_4$-Alkoxy, $C_1$-$C_2$-Halogenalkyl, $C_1$-$C_2$-Halogenalkoxy, gegebenenfalls substituiertes Phenyl, gegebenenfalls substituiertes Phenoxy, gegebenenfalls substituiertes Benzyl oder gegebenenfalls substituiertes Benzyloxy bedeutet, oder in der die Gruppe

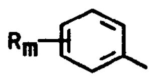

α-Naphthyl oder β-Naphthyl bedeutet und

X

Methylenoxy, Oxymethylen, Ethylen, Ethenylen, Thiomethylen oder Sauerstoff bedeutet.

3. Fungizid, enthaltend einen inerten Trägerstoff und eine fungizid wirksame Menge einer Verbindung der Formel I

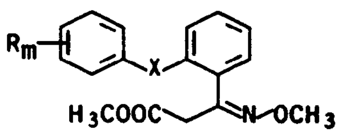

(I)

in der

R (m = 1 bis 5) gleiche oder verschiedene Substituenten Wasserstoff, Halogen, Cyano, Nitro, $C_1$-$C_{15}$-Alkyl, $C_3$-$C_6$-Cycloalkyl, $C_3$-$C_6$-Alkenyl, $C_1$-$C_4$-Alkoxy, $C_1$-$C_2$-Halogenalkyl, $C_1$-$C_2$-Halogenalkoxy, gegebenenfalls substituiertes Phenyl, gegebenenfalls substituiertes Phenoxy, gegebenenfalls substituiertes Benzyl oder gegebenenfalls substituiertes Benzyloxy bedeutet, oder in der die Gruppe

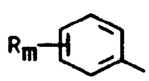

α-Naphthyl oder β-Naphthyl bedeutet und

X

Methylenoxy, Oxymethylen, Ethylen, Ethenylen, Thiomethylen oder Sauerstoff bedeutet.

4. Verfahren zur Herstellung eines fungiziden Mittels, dadurch gekennzeichnet, daß man eine oder mehrere Verbindungen der Formel I gemäß Anspruch 1 mit einem festen oder flüssigen Trägerstoff sowie gegebenenfalls mit einem oder mehreren oberflächenaktiven Mitteln mischt.

5. Verfahren zur Herstellung von 3-Methoximinopropionsäureestern der Formel I gemäß Anspruch 1, dadurch gekennzeichnet, daß man einen β-Keto-Carbonsäureester der allgemeinen Formel II

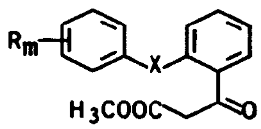

(II)

in der
R (m = 1 bis 5) gleiche oder verschiedene Substituenten Wasserstoff, Halogen, Cyano, Nitro, $C_1$-$C_{15}$-Alkyl, $C_3$-$C_6$-Cycloalkyl, $C_3$-$C_6$-Alkenyl, $C_1$-$C_4$-Alkoxy, $C_1$-$C_2$-Halogenalkyl, $C_1$-$C_2$-Halogenalkoxy, gegebenenfalls substituiertes Phenyl, gegebenenfalls substituiertes Phenoxy, gegebenenfalls substituiertes Benzyl oder gegebenenfalls substituiertes Benzyloxy bedeutet, oder in der die Gruppe

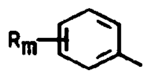

α-Naphthyl oder β-Naphthyl bedeutet und
X
Methylenoxy, Oxymethylen, Ethylen, Ethenylen, Thiomethylen oder Sauerstoff bedeutet, mit O-Methylhydroxylamin oder Hydroxylamin und einem Methylierungsmittel umsetzt.

6. β-Ketoester der Formel II

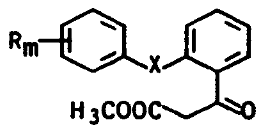

(II)

in der
R (m = 1 bis 5) gleiche oder verschiedene Substituenten Wasserstoff, Halogen, Cyano, Nitro, $C_1$-$C_{15}$-Alkyl, $C_3$-$C_6$-Cycloalkyl, $C_3$-$C_6$-Alkenyl, $C_1$-$C_4$-Alkoxy, $C_1$-$C_2$-Halogenalkyl, $C_1$-$C_2$-Halogenalkoxy, gegebenenfalls substituiertes Phenyl, gegebenenfalls substituiertes Phenoxy, gegebenenfalls substituiertes Benzyl oder gegebenenfalls substituiertes Benzyloxy bedeutet, oder in der die Gruppe

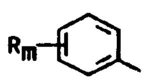

α-Naphthyl oder β-Naphthyl bedeutet und
X
Methylenoxy, Oxymethylen, Ethylen, Ethenylen, Thiomethylen oder Sauerstoff bedeutet.

7. Verbindung der Formel I gemäß Anspruch 1, in der $R_m$ 2-Methyl und X Methylenoxy bedeutet.

8. Verbindung der Formel I gemäß Anspruch 1, wobei $R_m$ 4-Fluor und X Methylenoxy bedeutet.

9. Verbindung der Formel I gemäß Anspruch 1, wobei $R_m$ 4-Chlor und X Methylenoxy bedeutet.

## Claims

1. A 3-methoximinopropionic ester of the formula I

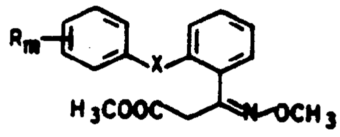

(I),

where the radicals R (m = 1 to 5) are identical or different and are each hydrogen, halogen, cyano, nitro, $C_1$-$C_{15}$-alkyl, $C_3$-$C_6$-cycloalkyl, $C_3$-$C_6$-alkenyl, $C_1$-$C_4$-alkoxy, $C_1$- or $C_2$-haloalkyl, $C_1$- or $C_2$-haloalkoxy, unsubstituted or substituted phenyl, unsubstituted or substituted phenoxy, unsubstituted or substituted benzyl or unsubstituted or substituted benzyloxy, or the group

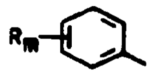

is α-naphthyl or β-naphthyl, and X is methyleneoxy, oxymethylene, ethylene, ethenylene, thiomethylene or oxygen.

2. A method for controlling fungi, wherein the fungi, or the materials, plants, seed or soil threatened by fungus, attack are treated with a fungicidally effective amount of a compound of the formula I

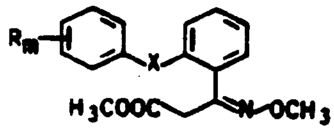

(I),

where the radicals R (m = 1 to 5 ) are identical or different and are each hydrogen, halogen, cyano, nitro, $C_1$-$C_{15}$-alkyl, $C_3$-$C_6$-cycloalkyl, $C_3$-$C_6$-alkenyl, $C_1$-$C_4$-alkoxy, $C_1$-$C_2$-haloalkyl, $C_1$-$C_2$-haloalkoxy, unsubstituted or substituted phenyl, unsubstituted or substituted phenoxy, unsubstituted or substituted benzyl or unsubstituted or substituted benzyloxy, or the group

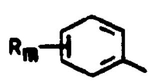

is α-naphthyl or β-naphthyl, and X is methyleneoxy, oxymethylene, ethylene, ethenylene, thiomethylene or oxygen.

3. A fungicide containing an inert carrier and a fungicidally effective amount of a compound of the formula I

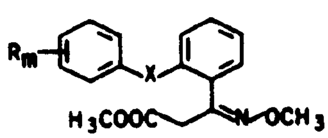

(I),

where the radicals R (m = 1 to 5) are identical or different and are each hydrogen, halogen, cyano, nitro, $C_1$-$C_{15}$-alkyl, $C_3$-$C_6$-cycloalkyl, $C_3$-$C_6$-alkenyl, $C_1$-$C_4$-alkoxy, $C_1$-$C_2$-haloalkyl, $C_1$-$C_2$-haloalkoxy, unsubstituted or substituted phenyl, unsubstituted or substituted phenoxy, unsubstituted or substituted benzyl or unsubstituted or substituted benzyloxy, or the group

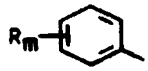

is α-naphthyl or β-naphthyl, and X is methyleneoxy, oxymethylene, ethylene, ethenylene, thiomethylene or oxygen.

4. A process for the preparation of a fungicide, wherein one or more compounds of the formula I as claimed in claim 1 are mixed with a solid or liquid carrier and, if required, with one or more surfactants.

5. A process for the preparation of a 3-methoximinopropionic ester of the formula I as claimed in claim 1, wherein a β-keto carboxylic ester of the formula (II)

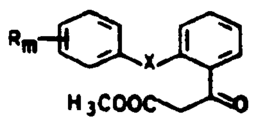

(II)

where the radicals R (m = 1 to 5) are identical or different and are each hydrogen, halogen, cyano, nitro, $C_1$-$C_{15}$-alkyl, $C_3$-$C_6$-cycloalkyl, $C_3$-$C_6$-alkenyl, $C_1$-$C_4$-alkoxy, $C_1$- or $C_2$-haloalkyl, $C_1$- or $C_2$-haloalkoxy, unsubstituted or substituted phenyl, unsubstituted or substituted phenoxy, unsubstituted or substituted benzyl or unsubstituted or substituted benzyloxy, or the group

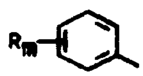

is α-naphthyl or β-naphthyl, and X is methyleneoxy, oxymethylene, ethylene, ethenylene, thiomethylene or oxygen, is reacted with O-methylhydroxylamine or hydroxylamine and a methylating agent.

6. A β-ketoester of the formula II

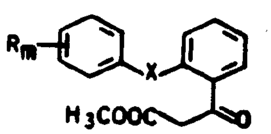

(II)

where the radicals R (m = 1 to 5) are identical or different and are each hydrogen, halogen, cyano, nitro, $C_1$-$C_{15}$-alkyl, $C_3$-$C_6$-cycloalkyl, $C_3$-$C_6$-alkenyl, $C_1$-$C_4$-alkoxy, $C_1$- or $C_2$-haloalkyl, $C_1$- or $C_2$-haloalkoxy, unsubstituted or substituted phenyl, unsubstituted or substituted phenoxy, unsubstituted or substituted benzyl or unsubstituted or substituted benzyloxy, or the group

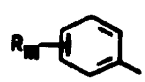

is α-naphthyl or β-naphthyl, and X is methyleneoxy, oxymethylene, ethylene, ethenylene, thiomethylene or oxygen.

7. A compound of the formula I as claimed in claim 1, where $R_m$ is 2-methyl and X is methyleneoxy.

8. A compound of the formula I as claimed in claim 1, where $R_m$ is 4-fluoro and X is methyleneoxy.

9. A compound of the formula I as claimed in claim 1, where $R_m$ is 4-chloro and X is methyleneoxy.

## Revendications

1. Esters 3-méthoximinopropioniques de formule I

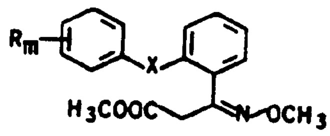

(I)

dans laquelle
les symboles R (m ayant une valeur de 1 à 5), ayant des significations identiques ou différentes, représentent chacun l'hydrogène, un halogène, un groupe cyano, nitro, alkyle en C1-C15, cycloalkyle en C3-C6, alcényle en C3-C6, alcoxy en C1_C4, halogénoalkyle en C1-C2, halogénoalcoxy en C1-C2, phényle éventuellement substitué, phénoxy éventuellement substitué, benzyle éventuellement substitué ou benzyloxy éventuellement substitué, ou bien dans laquelle

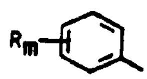

représente un groupe alpha-naphtyle ou bêta-naphtyle, et
X représente un groupe méthylène-oxy, oxyméthylène, éthylène, éthénylène, thiométhylène ou l'oxygène.

2. Procédé pour combattre les mycètes, caractérisé en ce que l'on traite les mycètes ou les matières, végétaux, semences ou le sol menacés d'une attaque par les mycètes par une quantité fongicide efficace d'un composé de formule I

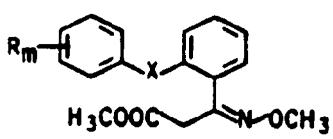

(I)

dans laquelle
les symboles R (m ayant une valeur de 1 à 5), ayant des significations identiques ou différentes, représentent chacun l'hydrogène, un halogène, un groupe cyano, nitro, alkyle en C1-C15, cycloalkyle en C3-C6, alcényle en C3-C6, alcoxy en C1-C4, halogénoalkyle en C1-C2, halogénoalcoxy en C1-C2, phényle éventuellement substitué, phénoxy éventuellement substitué, benzyle éventuellement substitué ou benzyloxy éventuellement substitué, ou bien dans laquelle

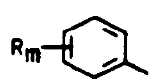

représente un groupe alpha-naphtyle ou bêta-naphtyle, et
X représente un groupe méthylène-oxy, oxyméthylène, éthylène, éthénylène, thiométhylène ou l'oxygène.

3. Produit fongicide contenant un véhicule inerte et une quantité fongicide efficace d'un composé de formule I

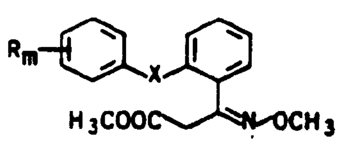

(I)

dans laquelle
les symboles R (m ayant une valeur de 1 à 5), ayant des significations identiques ou différentes, représentent chacun l'hydrogène, un halogène, un groupe cyano ou nitro, alkyle en C1-C15, cycloalkyle en C3-C6, alcényle en C3-C6, alcoxy en C1-C4, halogénoalkyle en C1-C2, halogénoalcoxy en C1-C2, phényle éventuellement substitué, phénoxy éventuellement substitué, benzyle éventuellement substitué ou benzyloxy éventuellement substitué, ou dans laquelle

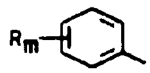

représente un groupe alpha-naphtyle ou bêta-naphtyle, et
X représente un groupe méthylène-oxy, oxyméthylène, éthylène, éthénylène, thiométhylène ou l'oxygène.

4. Procédé de préparation d'un produit fongicide, caractérisé en ce que l'on mélange un ou plusieurs composés de formule I de la revendication 1 avec un véhicule solide ou liquide et le cas échéant un ou plusieurs agents tensioactifs.

5. Procédé de préparation des esters 3-méthoximinopropioniques de formule I de la revendication 1, caractérisé en ce que l'on fait réagir un ester bêta-cétocarboxylique de formule générale II

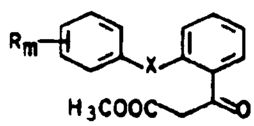

(II)

dans laquelle
les symboles R (m ayant une valeur de 1 à 5), ayant des significations identiques ou différentes, représentent chacun l'hydrogène, un halogène, un groupe cyano, nitro, alkyle en C1-C15, cycloalkyle en C3-C6, alcényle en C3-C6, alcoxy en C1-C4, halogénoalkyle en C1-C2, halogénoalcoxy en C1-C2, phényle éventuellement substitué, phénoxy éventuellement substitué, benzyle éventuellement substitué ou benzyloxy éventuellement substitué, ou bien dans laquelle

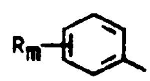

représente un groupe alpha-naphtyle ou bêta-naphtyle, et
X représente un groupe méthylène-oxy, oxyméthylène, éthénylène, thiométhylène ou l'oxygène, avec l'O-méthylhydroxylamine ou l'hydroxylamine et un agent méthylant.

6. Bêta-cétoesters de formule II

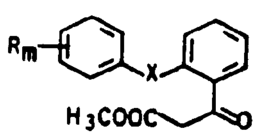

(II)

dans laquelle
les symboles R (m ayant une valeur de 1 à 5 , ayant des significations identiques ou différentes, représentent chacun l'hydrogène, un halogène, un groupe cyano, nitro, alkyle en C1-C15, cycloalkyle en C3-C6, alcényle en C3-C6, alcoxy en C1-C4, halogénoalkyle en C1-C2, halogénoalcoxy en C1-C2, phényle éventuellement substitué, phénoxy éventuellement substitué, benzyle éventuellement substitué ou benzyloxy éventuellement substitué, ou bien dans laquelle

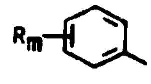

représente un groupe alpha-naphtyle ou bêta-naphtyle, et
X représente un groupe méthylène-oxy, oxyméthylène, éthylène, éthénylène, thiométhylène ou l'oxygène.

**7.** Composé de formule I de la revendication 1 dans laquelle $R_m$ représente un groupe 2-méthyle et X un groupe méthylène-oxy.

**8.** Composé de formule I de la revendication 1, dans laquelle $R_m$ représente un substituant 4-fluoro et X un groupe méthylène-oxy.

**9.** Composé de formule I de la revendication 1, dans laquelle $R_m$ représente un substituant 4-chloro et X un groupe méthylène-oxy.